# EUROPEAN PATENT SPECIFICATION

(11) **EP 3 183 555 B1**
(45) Date of publication and mention of the grant of the patent: **04.08.2021**
(21) Application number: 15833910.1
(22) Date of filing: 20.08.2015
(51) Int. Cl.: G01N 15/02, G01N 21/51, G01N 21/53, G01N 21/05, G01N 21/64, C12Q 3/00, G01N 15/06, G01N 15/00

(54) **DEVICES, SYSTEMS AND METHODS FOR DETECTING PARTICLES**
VORRICHTUNGEN, SYSTEME UND VERFAHREN ZUR ERKENNUNG VON PARTIKELN
DISPOSITIFS, SYSTÈMES ET PROCÉDÉS DE DÉTECTION DE PARTICULES

(30) Priority: 20.08.2014 US 201462039512 P; 20.08.2014 US 201462039519 P
(43) Date of publication of application: 28.06.2017
(73) Proprietor: Research Triangle Institute, Research Triangle Park, NC 27709 (US)
(72) Inventor: CLAYTON, Anthony Clint, Research Triangle Park, NY 27709 (US); WALLS, Howard Jerome, Research Triangle Park, NY 27709 (US); NEWSOME, Randall J., Research Triangle Park, NY 27709 (US); HOERTZ, Paul G., Research Triangle Park, NY 27709 (US)
(74) Representative: Mollekopf, Gerd Willi
(86) International application number: PCT/US2015/046076
(87) International publication number: WO 2016/028996

(56) References cited:
- WO-A1-01/95279
- WO-A1-90/10858
- WO-A1-2005/001436
- CN-A- 103 983 544
- US-A- 3 849 654
- US-A1- 2009 153 857
- WALLACE, LANCE: 'Real-time measurements of black carbon indoors and outdoors: a comparison of the photoelectric aerosol sensor and the aethalometer, Aerosol' SCIENCE AND TECHNOLOGY vol. 39, no. 10, 01 January 2005, pages 1015 - 1025, XP055407670 DOI: 10.1080/02786820500365363
- PAHALAWATTA ET AL.: 'Particle detection and classification in photoelectric smoke detectors using image histogram features.' 2013 INTERNATIONAL CONFERENCE ON DIGITAL IMAGE COMPUTING: TECHNIQUES AND APPLICATIONS(DICTA) 26 November 2013, pages 1 - 8, XP032536444 DOI: 10.1109/DICTA.2013.6691509
- None

## Description

### TECHNICAL FIELD

The present invention relates to optical-based detection of particles in an aerosol or liquid, including measurement of light scattering and autofluorescence.

### BACKGROUND

Detection of particles and colloids suspended in a fluid medium for measurement of concentration or other properties is useful in a variety of applications such as medical diagnostics, scientific research, air quality measurements, and threat detection. Examples include measurement of the concentration of particles suspended in a liquid such as proteins in blood, and airborne particles in inside environments such as building as well as outside environments.

One application of note is the measurement of the concentration and other properties of airborne particles (or particulate matter, PM) in aerosols. The United States Environmental Protection Agency (US EPA) has set exposure standards for coarse PM (between 10 µm and 2.5 µm, PM₁₀) and fine PM (less than 2.5 µm, PM_{2.5}) due to the importance of aerosol concentration in the air and its health effects. Aerosol concentrations are also important in the manufacturing industry for both protection of the health of workers and preventing contamination in the manufacturing process.

A class of aerosols of special interest is bioaerosols. Bioaerosols include bio-particles such as fungus spores, bacteria spores, bacteria, viruses, and biologically derived particles (skin cells, detritus, etc.). Some bioaerosols cause chronic and/or acute health effects, for example certain strains of black mold or *Bacillus anthraces* (causative bacteria of anthrax). Bioaerosol concentrations are important in maintaining safe hospitals, clean food processing, pharmaceutical and medical device manufacturing, and air quality. Airborne spread of diseases is of particularly concern from a public health perspective. Aerosolized bioagents can also be used by terrorists to harm civilian or military populations.

Measurement (sensing) of aerosol and bioaerosol concentration is typically accomplished with optical techniques. Aerosol (e.g., solid and liquid particles ≤10 µm dispersed in air) concentration measurement is readily achieved by various light scattering measurements. *See* Hinds, Aerosol Technology, New York, John Wiley & Sons, Inc. (1982); Lehtimaki and Willeke, Measurement Methods, Aerosol Measurement, Willeke and Baron, New York, Van Norstrand Reinhold, 112-129 (1993). The most accurate method entails the use of a single particle counter that focuses a stream of aerosol into a detection cavity where light scattering from a long wavelength (> 650 nm) laser is measured. Precision optics are required to collect and focus the scattered light (while excluding the source light) onto a photon detector. The photon detectors are made from silicon or photocathode materials (e.g., indium gallium arsenide) that undergo the photoelectric effect (convert photons to electrons). These materials are packaged into detectors that offer high amplification of the signal from the photons, such as photomultiplier tubes (PMTs) and avalanche photodiodes (APDs). These detectors have active detection areas that are small (less than 25 mm²) and limited to planar geometries. Moreover, these detectors cost $100 or more, often exceeding $1,000 in the case of a high sensitivity PMT.

Autofluorescence (or intrinsic fluorescence) excited by ultraviolet (UV) and blue light is well-developed for detection of bioaerosols. *See* Hairston et al., "Design of an instrument for real-time detection of bioaerosols using simultaneous measurement of particle aerodynamic size and intrinsic fluorescence," Journal of Aerosol Science 28(3): 471-482 (1997); Ho, "Future of biological aerosol detection," Analytical Chimica Acta 457(1): 125-148 (2002); Agranovski et al., "Real-time measurement of bacterial aerosols with the UVAPS: Performance evaluation," Journal of Aerosol Science 34(3): 301-317 (2003); Ammor, "Recent advances in the use of intrinsic fluorescence for bacterial identification and characterization," Journal of Fluorescence 17(5): 455-459 (2007); Ho et al., "Feasability of using real-time optical methods for detecting the presence of viable bacteria aerosols at low concentrations in clean room environments," Aerobiologia 27(2): 163-172 (2011). Exploiting autofluorescence of microbes is widely viewed as one of the most cost-effective means to detect a potential biological threat. Bioaerosol detectors typically use a combination of light scattering (measurement of general aerosol concentration and properties) and autofluorescence (detection of emitted photons). Bioaerosol detectors based on autofluorescence rely on fluorescence from molecular fluorophores that reside within the bio-particle. For clean bio-particles, this fluorescence can be primarily attributed to biochemicals such as tryptophan and tyrosine (amino acids), nicotinamide adenine dinucleotide (NADH), and riboflavin. NADH and riboflavin absorb and emit longer wavelengths than the amino acids. *See* Jeys et al., "Advanced trigger development," Lincon Laboratory Journal 17(1): 29-62 (2007); Hill et al., "Fluorescence of bioaerosols: mathematical model including primary fluorescing and absorbing molecules in bacteria," Optics Express 21(19): 22285-22313 (2013). The ability to use longer wavelength excitation sources such as light-emitting diodes (LEDs, excitation wavelength λ_{exc} > 360 nm) or lasers (λ_{exc} > 400 nm) may reduce the cost of such instruments.

Traditional bioaerosol particle detectors rely on three main components: (1) an excitation source of appropriate wavelength to excite a targeted fluorophore or collection of fluorophores; (2) precision optics (lenses and mirrors) on both the excitation and emission side to focus the source onto the narrow air stream and to enhance the collection of emitted photons from biological particles; and (3) a high gain detector such as a PMT or APD. Elastic light scattering from visible or long wavelengths is utilized to count and sometimes size the particles. Autofluorescence of biomolecules is utilized to detect microorganisms. The typical bioaerosol detector utilizes a small detection cavity, with fluorescence active volumes on the order of 1 × 10⁻⁴ cm³, making the window for detection of each bioaerosol particle exceedingly small. At typical flow rates, a bioaerosol particle resides within the excitation volume for 1-10 µs on average. *See* Hairston et al. (1997). As a result, emitted and scattered light from each bioaerosol particle is collected virtually on an individual basis, and the signal is weak. *See* Greenwood et al., "Optical Techniques for Detecting and Identifying Biological Warfare Agents," Proceedings of the IEEE 97(6): 971-989 (2009). This weak signal thus requires the use of precision lenses and mirrors to collect the weak signal and focus it onto the high gain detector (e.g., PMT or APD).

Measurement of aerosol and bioaerosol concentration and changes in concentration is possible via a variety of commercially available instruments such as the Laser Aerosol Spectrometer for aerosols (TSI Incorporated, Shoreview, Minnesota, USA), the Ultraviolet Aerodynamic Particle Sizer for bioaerosols (TSI Incorporated), the Wideband Integrated Bioaerosol Sensor (WIBS-4) for bioaerosols (Droplet Measurement Technologies, Boulder, Colorado, USA), and the instantaneous biological analyzer and collector (FLIR Systems, Inc., Wilsonville, Oregon, USA). However, such instruments can exceed $10,000 in cost making wide spread use cost prohibitive. Furthermore, having a sufficiently dense sensor network of aerosol/bioaerosol sensors (i.e., multiples of these instruments in communication with a central network) is cost prohibitive. The high cost of a sensor network also means that capitalizing on responsive systems is challenging. For example, it would be desirable to provide several bioaerosol sensors positioned throughout a hospital or other building and networked with the building's control systems to maintain a safe environment and respond to a change in bioaerosol concentration, such as by diverting airflow or indicating the need for maintenance of filters and air handlers.

Aerosol exposure monitors have been developed that acquire data from aerosol while the aerosol is sampled in real time during a prescribed sampling period (integration period). Such devices may employ inertial impactors for aerodynamic sizing, particle collection filters for collection and subsequent analysis, and nephelometers for measuring particle concentration by acquiring light scattering data in real time. Examples of such devices are described in International Publication No. WO 2013/063426, filed October 26, 2012, titled "AEROSOL EXPOSURE MONITORING". Also known are turbidometers, which measure the concentrations of particles such as cells in solution.

There is an ongoing need for devices and methods for measurement of particles in aerosols, bioaerosols, and liquids to obtain data relating to concentration and other properties of particles. There is also a need for such devices that operate effectively with simplified designs geometries, and a reduced number and cost of precision parts. There is also a need for such devices that require less cost and effort to fabricate, operate, and maintain such devices.

WO 2005/001436 A1 suggests a fluid borne particle analyzer where a quartz tube is guided through an integrating sphere (Ulbricht sphere) for homogenizing / integrating deflected light. The quartz tube has a fluid inlet and fluid outlet. Along the tube axis a light beam from a light source is guided. At a window of the integrating sphere an optical detector is arranged.

The particle detector of US 2009/0153857 A1 has a flow cell through which a sample fluid is flowing. A laser beam from a laser light source is directed along the axis of the flow cell and scattered light from within the flow cell is projected by a lens to the active surface of a photoelectric transducer element.

### SUMMARY

The invention is defined in claims 1 and 13, respectively. Particular embodiments are set out in the dependent claims.

The sample fluid may be an aerosol.

The sample fluid may be a liquid.

### BRIEF DESCRIPTION OF THE DRAWINGS

The invention can be better understood by referring to the following figures. The components in the figures are not necessarily to scale, emphasis instead being placed upon illustrating the principles of the invention. In the figures, like reference numerals designate corresponding parts throughout the different views.
Figure 1 is a schematic perspective view of an example of a particle detector according to some embodiments of the present disclosure.
Figure 2 is a schematic cross-sectional view (x-y plane) of the particle detector illustrated in Figure 1, taken at an arbitrary point along a longitudinal axis (z-axis).
Figure 3 is a schematic plan view of the particle detector illustrated in Figure 1, arbitrarily taken as the x-z plane.
Figure 4 is a schematic plan view of the particle detector illustrated in Figure 1, illustrating an example of a stray light blocking device that may be utilized in the particle detector.
Figure 5 is a schematic plan view of the particle detector illustrated in Figure 1, illustrating an example of beam shaping optics that may be utilized in the particle detector.
Figure 6 is a schematic plan view of an example of a flexible light detector that may be utilized in the particle detector illustrated in Figure 1.
Figure 7 is a schematic cross-sectional view of the particle detector according to other embodiments.
Figure 8 is a schematic plan view of another example of a particle detector according to some embodiments.
Figure 9 is a schematic plan view of another example of a particle detector according to some embodiments.
Figure 10 is a schematic plan view of another example of a particle detector according to some embodiments.
Figure 11 is a schematic cross-sectional view of another example of a particle detector not forming part of the present invention.
Figure 12 is a plot of size distribution of ISO Ultrafine Arizona Road Dust as measured with a Scanning Mobility Particle Sizer (SMPS) and sampled by a particle detector as described below in Example 1; the Dust was dispersed into an aerosol mixing chamber and then sampled with the SMPS connected to a Condensation Particle Counter (CPC).
Figure 13A is a plot of aerosol concentration, in which an aerosol mixing chamber is charged with a quantity of Ultrafine Arizona Road Dust and then sampled by the particle detector described below in Example 1, an Aerodynamic Particle Sizer (APS), and the CPC referred to above in conjunction with Figure 12; the decay in aerosol concentration in the chamber is captured by all three instruments; the particle detector response reported is the measured voltage minus the baseline measured with the laser on but with particle free air flowing through the sensor.
Figure 13B is a plot comparing the response of the particle detector described below in Example 1 to the particle counting of the APS; the particle detector response reported is the measured voltage minus the baseline measured with the laser on but with particle free air flowing through the sensor.
Figure 13C is a plot comparing the response of the particle detector described below in Example 1 to the particle counting of the CPC; the particle detector response reported is the measured voltage minus the baseline measured with the laser on but with particle free air flowing through the sensor.
Figure 14 is a plot of the response of a particle detector as described below in Example 2 to Tinopal tagged and untagged Syloid aerosol as simulants for inert aerosol and bioaerosol; the response of a photovoltaic light detector of the particle detector protected by an ultraviolet UV gel filter is due to emitted fluorescence and not the scattered excitation photons.
Figure 15 is a plot of the response of a particle detector as described below in Example 2 to fluorescently tagged Syloid aerosol (2% Tinopal); an APS and CPC provide total particle count (do not measure fluorescence); the particle detector response reported is the measured voltage minus the baseline measured with the laser on but with particle free air flowing through the sensor.
Figure 16 is a plot of the response of a particle detector as described below in Example 3 with violet 405 nm and red 650 nm lasers for *Bg* spore aerosol.

### DETAILED DESCRIPTION

As used herein, the term "aerosol" generally refers to an assembly of liquid or solid particles (or particulates, or particulate matter) suspended in a gaseous medium long enough to be observed and measured. The size of aerosol particles typically ranges from about 0.001 µm to about 100 µm. *See* Kulkarni et al., Aerosol Measurement, 3rd ed., John Wiley & Sons, Inc. (2011), p. 821. The term "gaseous fluid" generally refers to a gas (or gaseous fluid, or gas-phase fluid). A gas may or may not contain liquid droplets or vapor, and may or may not contain aerosol particles. An example of a gas is, but is not limited to, ambient air. An aerosol may thus be considered as comprising particles and a gas that entrains or carries the particles.

As used herein, the term "bioaerosol" generally refers to an aerosol in which one or more bio-particles are suspended or carried. The term "bio-particle" generally refers to a biological material, or the combination of a biological material and a non-biological particle on which the biological material is carried. That is, a biological material may itself be a particle freely suspended in an aerosol, or may be carried on a non-biological particle such that the biological material and the non-biological particle are suspended together in the aerosol. The biological material may be carried on the non-biological particle by any mechanism such as, for example, entrapment, embedment, adhesion, adsorption, attractive force, affinity, etc. Examples of biological materials include, but are not limited to, spores (e.g., fungal spores, bacterial spores, etc.), fungi, molds, bacteria, viruses, biological cells or intracellular components, biologically derived particles (e.g., skin cells, detritus, etc.), etc.

As used herein, for convenience the term "aerosol" generally encompasses the term "bioaerosol" and the term "particle" generally encompasses the term "bio-particle," unless indicated otherwise or the context dictates otherwise.

As used herein, the term "fluid" generally encompasses the term "liquid" as well as term "gas," unless indicated otherwise or the context dictates otherwise. Particles suspended or carried in a liquid, as well as particles suspended or carried in an aerosol, may be detected by devices and methods disclosed herein.

As used herein, the term "light" generally refers to electromagnetic radiation, quantizable as photons. As it pertains to the present disclosure, light may propagate at wavelengths ranging from ultraviolet (UV) to infrared (IR). In the present disclosure, the terms "light," "photons," and "radiation" are used interchangeably.

As used herein, a material is "optically transparent" if it is able to efficiently pass (with minimal optical transmission loss) light of a desired wavelength or range of wavelengths.

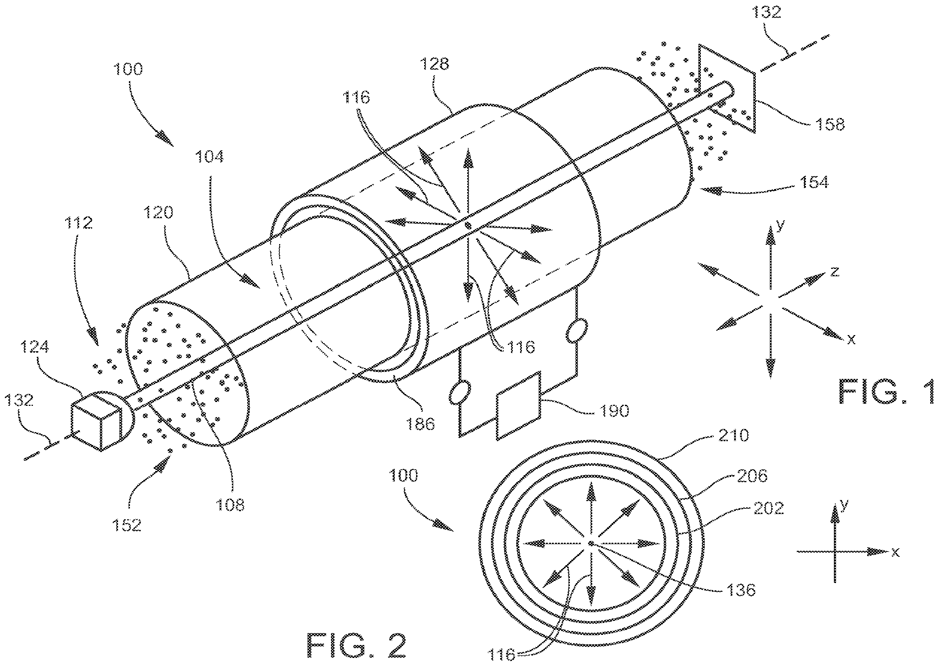

Figure 1 is a schematic perspective view of an example of a particle detector 100 according to some embodiments. Generally, the particle detector 100 is configured for defining (e.g., containing or enclosing) a detection cavity 104 (or sample volume) through which a particle-laden sample fluid (i.e., aerosol or liquid) may flow, producing one or more beams 108 of irradiating light (or source light) of one or more selected wavelengths, directing the beam(s) 108 into the detection cavity 104 to enable particles 112 in the detection cavity 104 to interact with the irradiating light incident on the particles 112, and collecting (receiving) measurement light (or emission light) emitted from the particles 112 in response to the irradiation. The particle detector 100 is configured for collecting measurement light over a large detection area (i.e., a large photon collection area), via a plurality of paths 116 over which the measurement light propagates, as partially depicted by rays in Figure 1. For these purposes, the particle detector 100 may include a housing 120 or other structure for defining a flow-through detection cavity 104, one or more light sources 124 for producing one or more beams 108 of irradiating light, and one or more light detectors 128 for collecting measurement light over a plurality of different paths 116. The particle detector 100 may be operated to acquire particle data in real time as sample fluid flows through the particle detector 100.

In the present context, "irradiating" light refers to the light produced by the light source 124 and utilized to irradiate particles in the detection cavity 104, as distinguished from measurement light and as also distinguished from background light (i.e., non-analytical light that would only contribute to background signal noise, such as ambient light). In the present context, "measurement" light refers to the light emitted from the particles in response to the irradiation. Measurement light may be light scattered (reflected) from the particles or fluorescent light emitted from the particles. The particle detector 100 may be configured for measuring scattered light and/or fluorescently emitted light. The particle detector 100 may be configured for measuring scattered light and fluorescently emitted light simultaneously or sequentially.

As regards scattered light, the particle detector 100 may be configured in particular for measuring elastically scattered light. Irradiating light incident on a particle may be elastically scattered from the particle at the same wavelength as the irradiating light, in accordance with the particle's size and shape and the difference in the index of refraction of the particle and that of the sample fluid. The scattering mode may be Rayleigh scattering, Mie scattering, or geometric scattering, depending on the size of the particle relative to the wavelength of the irradiating light. As regards fluorescently emitted light, the irradiating light may be utilized as an excitation light for inducing autofluorescence in the fluorophores of a particle (particularly a bio-particle). That is, irradiating light of an appropriate wavelength or wavelength range incident on a fluorophore-containing particle may be absorbed by the particle and thereby induce the particle to fluoresce, i.e., emit light at a different (typically longer) wavelength or wavelength range.

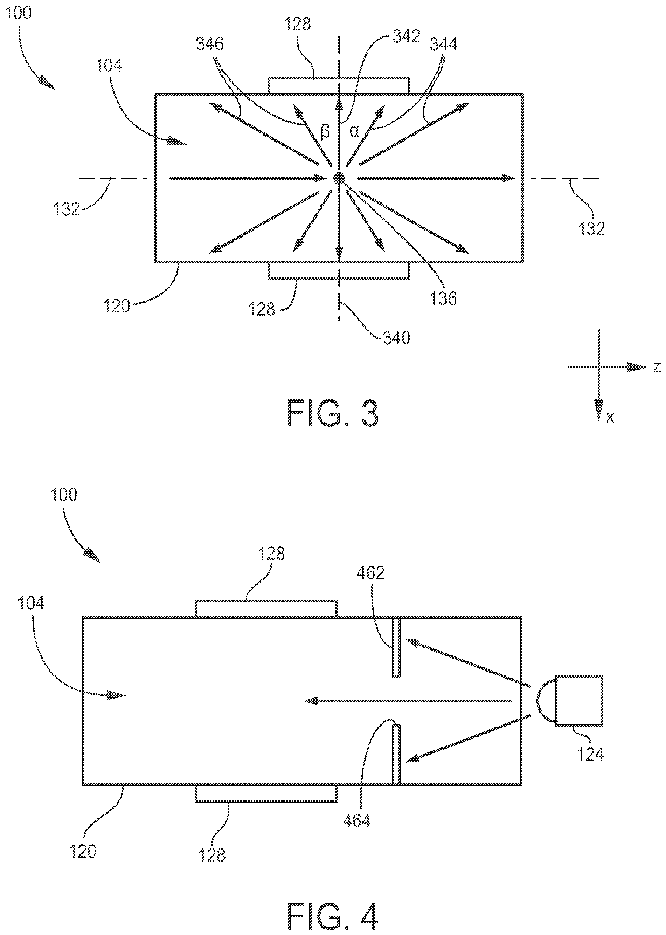

Generally, measurement light may propagate from an irradiated particle in any of a large number of directions relative to a longitudinal axis 132, as further shown in Figures 2 and 3. For reference purposes, the longitudinal axis 132 may be considered as the z-axis, and the cross-sectional plane orthogonal to the longitudinal axis 132 may be considered as the x-y plane. Figure 2 is a schematic cross-sectional view (x-y plane) of the particle detector 100 taken at an arbitrary point along the longitudinal axis 132. An irradiated particle 136 has been arbitrarily located directly on the longitudinal axis 132. As shown in Figure 2, most or all paths 116 along which the measurement light propagates have a radial component relative to the longitudinal axis 132. Figure 3 is a schematic plan view of the particle detector 100. The plan view has been arbitrarily taken as the x-z plane, with the understanding that rotating the particle detector 100 ninety degrees about the longitudinal axis 132 to the y-z plane would yield essentially the same view. The x-y plane in which the irradiated particle lies at the instant of time at or shortly after irradiation is indicated by a vertical dashed line 340. As shown in Figure 3, the paths or directions along which the measurement light propagates may include purely radial paths 342, forward-angle paths 344, and back-angle paths 346, relative to the x-y plane 340. In the present context, a purely radial path lies 342 substantially in the x-y plane 340, a forward-angle path 344 is oriented at some positive angle α relative to x-y plane 340 (i.e., has both a radial component and an axial component pointed in the downstream direction), and a back-angle path 346 is oriented at some negative angle β relative to x-y plane 340 (i.e., has both a radial component and an axial component pointed in the upstream direction). As described further below, the light detector 128 is capable of capturing photons propagating over a large number of purely radial paths 342, forward-angle paths 344, and back-angle paths 346 emanating from an irradiated particle 136.

Referring again to Figure 1, the housing 120 or other structure defining the detection cavity 104 may surround or enclose a chamber or interior about the longitudinal axis 132. The chamber or interior may be coextensive with, or at least may include, the detection cavity 104. The housing 120 (or a portion thereof defining the detection cavity 104) may be generally symmetrical about the longitudinal axis 132 such that the longitudinal axis 132 is the central axis of the housing 120 (or housing portion defining the detection cavity 104). In some embodiments, the housing 120 (or housing portion defining the detection cavity 104) may be generally cylindrical as illustrated in Figure 1, while in other embodiments may be spherical or polygonal. The housing 120 may be configured such that the detection cavity 104 is elongated along the longitudinal axis 132. As one example of an elongated geometry, the length of the detection cavity 104 along the longitudinal axis 132 may be greater than its cross-sectional dimension. In the present context, the term "cross-sectional dimension" refers to the maximum dimension that characterizes the size of the detection cavity's cross-section (cross-sectional flow area) in the plane orthogonal to the longitudinal axis 132 (e.g., the diameter of a circular cross-section, the major axis of an elliptical cross-section, or the length of a side or distance between opposing corners of a polygonal cross-section). The housing 120 includes a sample inlet 152 and a sample outlet 154 positioned such that the housing 120 defines a sample flow path from the sample inlet 152, through the detection cavity 104, and to the sample outlet 154. The sample inlet 152 and sample outlet 154 are typically open to the ambient environment outside the particle detector 100. The axial length of the detection cavity 104 may defined between a first end into which sample fluid is received and an axially opposite second end from which sample fluid is discharged. Depending on the configuration of the housing 120, the first end of the detection cavity 104 may generally correspond to (or be located proximal to) the sample inlet 152, and the second end of the detection cavity 104 may generally correspond to (or be located proximal to) the sample outlet 154.

The light source(s) 124 may be any light source suitable for producing irradiating light of a selected wavelength. Typically, the selected wavelength is a single wavelength, which may be a predominant wavelength or peak wavelength (or center wavelength) in a case where the light source 124 emits photons in a narrow wavelength band around the selected wavelength. The irradiating wavelength or wavelengths may be selected for implementing a certain type of measurement, such as scattered light or fluorescent light. Examples of light sources 124 include, but are not limited to, light emitting diodes (LEDs), lasers, laser diodes (LDs), and lamps configured for emitting light predominantly at a peak or center wavelength. The power at which the light source 124 emits irradiating light may be on the order of watts (e.g., 0.5 to 10 W), although more generally no limitation is placed on the output power of the light source 124. The light source 124 may be configured for continuous wave (CW) and/or pulsed operation. The light source 124 may be positioned relative to the detection cavity 104 such that the beam 108 of irradiation light is coaxial or substantially coaxial with the longitudinal axis 132. The light source 124 may be mounted to the housing 120 or other structure of the particle detector 100 by any suitable means. The light source 124 may be mounted at or proximal to the first end of the detection cavity 104, such that the irradiation light propagates generally parallel with and in the same direction as the sample fluid flows through the detection cavity. Depending on the type of light source 124 utilized, the beam 108 may be coherent or non-coherent (diverging). The beam 108 may provide a generally cylindrical particle irradiation region within the detection cavity 104 of large cross-section and thus large volume, as opposed to a line or point generated by a conventionally focused laser beam. The cross-section of the beam 108 may be circular or elliptical. The relatively large volume of the beam 108 may result in increased sensitivity and lowered limit of detection (LOD) of the particle detector 100. In some embodiments, the beam 108 has a cross-sectional dimension (e.g., diameter or major axis) in a range from 0.4 mm to 4 cm (4000 mm). In some embodiments, the beam 108 has a cross-sectional area in a range from 1% to 80% of the cross-sectional area of the detection cavity 104.

The light source 124 may be configured for emitting the irradiating light at an irradiating wavelength selected for the type of measurement to be made. In some embodiments, the irradiating wavelength is in a range from 250 to 1500 nm. In various embodiments, the irradiating wavelength may be in the ultraviolet range, the visible range, or the the infrared range. For measuring scattered light, the light source 124 may be selected based on factors such as low cost, emission at an irradiating wavelength that does not induce autofluorescence, etc. For measuring fluorescent emission, the light source 124 may be selected based on irradiating wavelength needed to excite certain bio-particles of interest. In some embodiments, longer irradiating wavelengths may be utilized for detecting scattered radiation while shorter irradiating wavelengths may be utilized for exciting fluorophores. For example, visible to long wavelengths such as violet (e.g., 405 nm) to infrared (IR, e.g., 900 nm) may be utilized for detecting scattered radiation, with red (e.g., 650 nm) to near IR wavelengths being typical in some embodiments. As another example, ultraviolet (UV) to blue wavelengths (e.g., 365 to 450 nm) may be utilized for exciting fluorophores. The TABLE below provides ground- and excited-state properties of a few biologically relevant fluorophores, nicotinamide adenine dinucleotide (NADH) and riboflavin, as well as an experimental surrogate, 2% Tinopal-on-Syloid, which is Syloid® silica powder (W.R. Grace and Company, Columbia, Maryland, USA) tagged with 2% Tinopal® CBS X florophore (BASF, Florham Park, New Jersey, USA).

**TABLE**

| Fluorophore | Total Fluorophores Per Particle, (#/particle) | Extinction Coefficient, (M⁻¹ cm⁻¹) | Absorbance Onset (nm) | Emission Spectral Range | Quantum Yield for Fluorescence | Fluorescence Lifetime (ns) |
|---|---|---|---|---|---|---|
| 2% Tinopal-on-Syloid | 1.5 x 10⁷ | 1,000 | < 420 | 380 - 575 | 0.81 | 1.2 |
| Free NADH (protein-bound NADH) | 4.8 x 10⁶ | 6,220 | <410 | 390 - 510 | 0.020 (0.08) | 0.38, 0.74 (1.2) |
| Riboflavin | 2 x 10⁶ | 15,000 | < 500 | 480 - 610 | 0.3 | 4.1 |

The particle detector 100 may include a light trap 158 (optical "beam dump") as shown for example in Figure 1. The light trap 158 may be positioned in optical alignment with the light source 124, on the opposite side of the detection cavity 104 as the light source 124. Generally, the light trap 158 may have any configuration suitable for effectively absorbing light and preventing light from being reflected back into the detection cavity 104. Various configurations for light traps are known to persons skilled in the art. As examples, the light trap 158 may include a plate or cavity that is opaque ("optically black") or anti-reflective, or at least the surface(s) of such plate or cavity facing the detection cavity 104 (or coating on the surface) is opaque or anti-reflective. The light trap 158 may include geometries or structures configured for trapping light as appreciated by persons skilled in the art. If needed, the light trap 158 may include a heat sink or other means for removing heat from the light trap 158.

If needed or desired, the particle detector 100 may include a device (one or more components) configured for preventing stray light from impinging on the light detector 128. Generally, stray light is any light having no analytical value such that measurement of the light by the light detector 128 is undesired. An example of stray light is irradiation light directly impinging on the light detector 128 without having first interacted with a particle to produce scattered or fluorescent light. Stray light elevates the detector output signal produced by the light detector 128 even in the absence of particles in the detection cavity 104, and thus may contribute to a large background (or baseline) signal that lowers the signal-to-noise (S/N) ratio of the particle detector 100, and may also convolute the measurement data. It is desirable to minimize the background signal to stay within the sensitive part of the response curve of the light detector 128. Testing has demonstrated that reducing the baseline voltage response of the light detector 128 from 1 volt (V) to a few millivolts (mV) dramatically lowered the LOD for aerosol from 1,000s #/cm³ to less than 100 #/cm³.

Figure 4 schematically illustrates an example of a device in the form of a plate 462 (or wall, baffle, etc.) having an aperture 464. Generally, the plate 462 may be located optically "downstream" of the light source 124, i.e., optically between the light source 124 and the light detector 128. The plate 462, or at least the surface of the plate 462 (or a coating on the surface) facing the light detector 128, may be opaque or anti-reflective to absorb irradiation light and any other stray light. Thus the plate 462 serves as a photon loss surface, blocking stray light that might otherwise reach the light detector 128. Meanwhile, the aperture 464 allows light (and sample fluid) to pass through the plate 462 along paths in the vicinity of the longitudinal axis 132, thereby ensuring that such light interacts with particles and is likely to be irradiation light of the intended wavelength. The axial position of the plate 462 relative to the light source 124 and the light detector 128, and the size of the aperture 464, may be selected as needed to optimize the photon-blocking function of the plate 462. The aperture 464 may be generally centered on the longitudinal axis 132. In some embodiments, the aperture 464 should be large enough that it does not act as a gas conductance barrier, cause localized turbulence, or otherwise appreciably modify the dynamics of the sample fluid flow through the detection cavity 104. More than one plate 462 may be provided if desired. Moreover, the plate 462 may include more than one aperture 464. In other embodiments, the beam 108 of irradiation light is sufficiently coherent and/or collimated that the plate 462 or similar device is not needed.

The housing 120, or at least the portion of the housing 120 defining the detection cavity 104, may be composed of a low reflectance material, or at least the inside surface of the housing 120 (or a coating applied thereon) may be composed of a low reflectance (or opaque, or anti-reflective) material. This may be useful in preventing stray light from reaching the light detector 128.

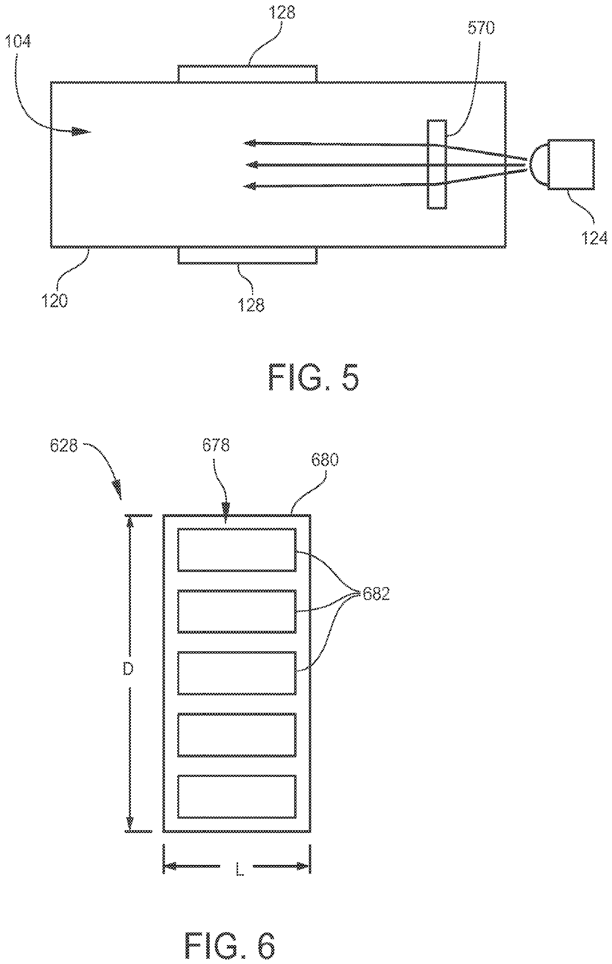

If needed or desired, the particle detector 100 may include beam shaping optics. The beam shaping optics may include one or more optics components (e.g., lenses). In the present context, the term "beam shaping optics" refers to an optical component that modifies a light beam or beam path without filtering out wavelengths. Figure 5 schematically illustrates an example of beam shaping optics 570. As one example, the beam shaping optics 570 may be or include a collimator (collimating lens) for collimating the beam of irradiation light. Such beam shaping optics 570 may be provided alternatively or in addition to the plate 462 or other stray light blocking device described above and illustrated in Figure 4. The axial position of the beam shaping optics 570 relative to the light source 124 may be selected as needed to optimize its beam shaping function. In other examples, the beam shaping optics 570 may be integrated into the package or assembly of the light source 124. In other examples, the beam 108 of irradiation light generated by the light source 124 is sufficiently coherent and/or collimated that a collimator separate and distinct from the light source 124 is not needed. As another example, in addition or as an alternative to a collimator, the beam shaping optics 570 may be or include a beam expander configured for increasing the diameter of the beam 108 emitted from the light source 124.

Referring to Figure 1, the light detector 128 is configured for collecting measurement light over a large detection area (i.e., a large photon collection area) via a plurality of paths 116 over which the measurement light propagates, including measurement light paths angled relative to the longitudinal axis 132 as described above. To this end, the light detector 128 may include a large-area active photoelectric material. The light detector 128 also includes one or more anodes and cathodes communicating with the active material as appreciated by persons skilled in the art. The light detector 128, or at least the photoelectric material, surrounds the detection cavity 104 along at least a portion of the cavity length. In the illustrated embodiment, the light detector 128 or at least the photoelectric material is constructed from a flexible material (one or more layers of flexible material(s)), enabling it either to be conformally wrapped around an outside surface of the housing 120 (or a portion of the housing 120 defining the detection cavity 104) or to conformally line an inside surface of the housing 120. In a typical embodiment, the photoelectric material is relatively thin so as to render it flexible (e.g., on the order of millimeters or smaller). The photoelectric material may be composed of any material (or composite of two or more materials) exhibiting efficient photoelectric activity and sufficiently sensitive over the range of wavelengths of measurement light contemplated for the particle detector 100. For example, the photoelectric material may be a thin-film inorganic, organic, or hybrid organic/inorganic semiconductor, one non-limiting example being amorphous silicon. The photoelectric material may generally be a material having at least one electrical characteristic (current, voltage, or resistance) that varies in proportion to light incident thereon.

In some embodiments, the photoelectric material is a photovoltaic (PV) material that produces both a current response and a voltage response to photons incident on its surface. For low light conditions, both a current response and voltage response are observed and are proportional to the amount of photons striking the PV material. The open-circuit voltage (OCV) of a PV material may show a measurable response to low-level particulate concentration changes (e.g., less than 100 #/cm³), due to the logarithmic response relationship between increases in low-level incident light (<< 0.1 Suns; or the amount of incident photons corresponding to elastic scattering from particles or fluorescence emissions) and the resulting increase in OCV. In other cases, such as high particle concentrations, measurement of the current response of the PV material may be more useful.

In a typical embodiment, at least one side of the photoelectric material is supported by a flexible substrate (e.g., a polymer layer or film such as polyimide). The photoelectric material may be completely encapsulated by (or embedded in) the substrate, or sandwiched between the substrate and an additional encapsulating layer or film, to protect the photoelectric material from the operating environment. Any layer or film covering the photon collecting side of the photoelectric material should be optically transparent. In some embodiments, the photon collecting side may be covered by a transparent electrode. The photon collecting side may be covered by a layer or film of an optical filter material, examples of which are described below.

The photoelectric material may completely or substantially completely surround the detection cavity 104 to provide a detection area spanning 360° or nearly 360° around the longitudinal axis 132. The photoelectric material may contiguously surround the detection cavity 104. Alternatively, the photoelectric material may include a plurality of discrete units or cells of photoelectric material spaced apart from each other and collectively surrounding the detection cavity 104.

Figure 6 is a schematic plan view of an example of a flexible light detector 628 that may be utilized in the particle detector 100. The light detector 628 includes a flexible photoelectric material 678 disposed on a flexible substrate 680. In this example, the photoelectric material 678 includes a plurality of photoelectric materials, or photoelectric units or cells 682. The photoelectric units 682 are spaced apart from each other, but may be closely grouped so as to maximize the size of the active detection area. While in the illustrated example the photoelectric units 682 are arranged in a one-dimensional array, in other embodiments they may be arranged in a two-dimensional array. The light detector 628 may initially be provided as a planar strip, and thereafter manipulated so as to surround the detection cavity 104. For example, the light detector 628 may be conformally mounted to the housing 120 as noted above. Thus, in the case of a cylindrical or spherical housing, the light detector 628 may surround the detection cavity 104 as a cylinder, band, or ring. The light detector 628 may present a significant surface area (L x D) largely occupied by the active material of the photoelectric units 682. As one non-limiting example, the dimension L may be on the order of one or more tens of millimeters, and the dimension D may be on the order of tens to hundreds of millimeters. When applied to a cylindrical or spherical housing, the dimensions L and D respectively correspond to a cylinder length and diameter of the light detector 628. The light detector 628 may include various current-carrying components (interconnects, wires, contacts, and the like, not shown) as appreciated by persons skilled in the art. In one non-limiting example, the light detector 628 may be based on a PV module commercially available from PowerFilm, Inc., Ames, Iowa, USA (e.g., model MP3-37).

In all such embodiments, the photoelectric material 678 provides a very large number of detection points surrounding the detection cavity 104 on which photons of the measurement light may be incident and thereby detected and measured. These detection points may be located at different angular positions relative to the central axis (over dimension D in Figure 6) and/or different axial positions relative to the longitudinal axis (over dimension L in Figure 6). As evident from Figures 2 and 3, the photoelectric material 678 provides a target for measurement light propagating over many different paths from an irradiated particle. By this configuration, the light detector 628 is able to output an electrical detector signal of relatively high intensity measurement even though individual optical measurement signals emanating from the particles may be relatively weak.

Referring to Figure 1, in some embodiments the particle detector 100 further includes one or more optical filters 186 positioned optically between the photon collecting side of the photoelectric material of the light detector 128 and the longitudinal axis 132. That is, the optical filter 186 is positioned such that any measurement light directed toward the photoelectric material must first pass through the optical filter 186. In some embodiments, the optical filter 186 is disposed on the photoelectric material, i.e., directly on the photoelectric material or on a layer or film covering or encapsulating the photoelectric material. The optical filter 186 generally may be configured to block one or more ranges of wavelengths, and thus may be a low-pass, high-pass, or band-pass filter. The optical filter 186 may be a composite of two or more optical filters to obtain the desired pass/block characteristics. The optical filter 186 may be a solid (e.g. glass or polymer) or gel (e.g. polymer) material, and may be thin and/or pliable enough to be flexible so as to conformally cover the photoelectric material. In one non-limiting example, a gel filter may be one commercially available from Rosco Laboratories, Inc., Stamford, Connecticut, USA.

The cross-sectional view of Figure 2 illustrates some examples of possible arrangements of the photoelectric material and optical filter relative to the housing. At the region of the detection cavity 104 where the photoelectric material and optical filter are located, the particle detector 100 may be considered as including at least three layers surrounding the detection cavity: a first (inner) layer 202, a second (intermediate) layer 206 surrounding the first layer 202, and a third (outer) layer 210 surrounding the second layer 206. In one embodiment, the first layer 202 is the optical filter, the second layer 206 is the housing (i.e., a wall of the housing), and the third layer 210 is the photoelectric material. Thus in this embodiment, the optical filter is conformally disposed on the inside surface of the housing, and the photoelectric material is conformally disposed on the outside surface of the housing. In another embodiment, the first layer 202 is the optical filter, the second layer 206 is the photoelectric material), and the third layer 210 is the housing. Thus in this embodiment, the photoelectric material is conformally disposed on the inside surface of the housing, and the optical filter is conformally disposed on the photoelectric material, such that the photoelectric material is sandwiched between the housing and the optical filter. In yet another embodiment, the first layer 202 is the housing, the second layer 206 is the optical filter, and the third layer 210 is the photoelectric material. Thus in this embodiment, the optical filter is conformally disposed on the outside surface of the housing, and the photoelectric material is conformally disposed on the optical filter, such that the optical filter is sandwiched between the photoelectric material and the housing. In cases where the photoelectric material is outside the housing, the housing (or at least the portion coextensive with the photoelectric material) is optically transparent. If needed, the layers 202, 206, and 210 may be secured to each other by any suitable means such as adhesives, mechanical fasteners, etc. In embodiments without the optical filter, the photoelectric material may be conformally disposed directly on the inside surface or outside surface of the housing.

The optical filter may generally be configured for blocking any selected wavelength or range(s) of wavelengths (undesired photons), depending on the application. For example, when measuring autofluorescence, the optical filter may be configured for passing the wavelengths of the fluorescent measurement light while blocking the wavelength of the irradiating light utilized to excite the fluorophores. As another example, when measuring scattering, the optical filter may be configured for passing the wavelength of the irradiating light (and thus the wavelength of the scattered measurement light) while blocking other wavelengths such as, for example, stray ambient light.

Referring to Figure 1, the particle detector 100 may further include a data acquisition device 190 that may be placed in signal communication with the light detector 128. The data acquisition device 190 may be configured for measuring a response of the photoelectric material (e.g., a voltage response, a current response, and/or resistance response), as embodied in an electrical detector signal outputted by the photoelectric material. The data acquisition device 190 may be configured for converting the analog detector signal to a digital detector signal, and recording or storing the detector signal. The data acquisition device 190 may be configured for correlating the measurement of the response with one or more properties of the particles interrogated by the irradiation light in the detection cavity 104, such as particle size, concentration, identification (e.g., a certain type of bio-particle), etc. The data acquisition device 190 may be configured for performing any post-acquisition signal conditioning or processing required or desired, such as amplification, calibration, deconvolution, formatting for transmission to another device, etc. The data acquisition device 190 may be configured for generating data relating to one or more properties of the interrogated particles, and transmitting the data to another device (e.g., a computing device) via a wired or wireless communication link, or to one or more devices via a suitable communication network. The data acquisition device 190 may be removably coupled to the light detector 124 such as by removable connections made with electrical leads from the photoelectric material. The data acquisition device 190 may thereafter be coupled to another device to download data to that other device for analysis. As appreciated by persons skilled in the art, various functions of the data acquisition device 190 may be implemented by hardware (or firmware), software, or both. The data acquisition device 190 may include one or more processors, memories, and other hardware. In one non-limiting example, the data acquisition device 190 may be a 16-bit data logging device commercially available from Measurement Computing Corp., Norton, Massachusetts, USA (e.g., model USB-1698FS-Plus).

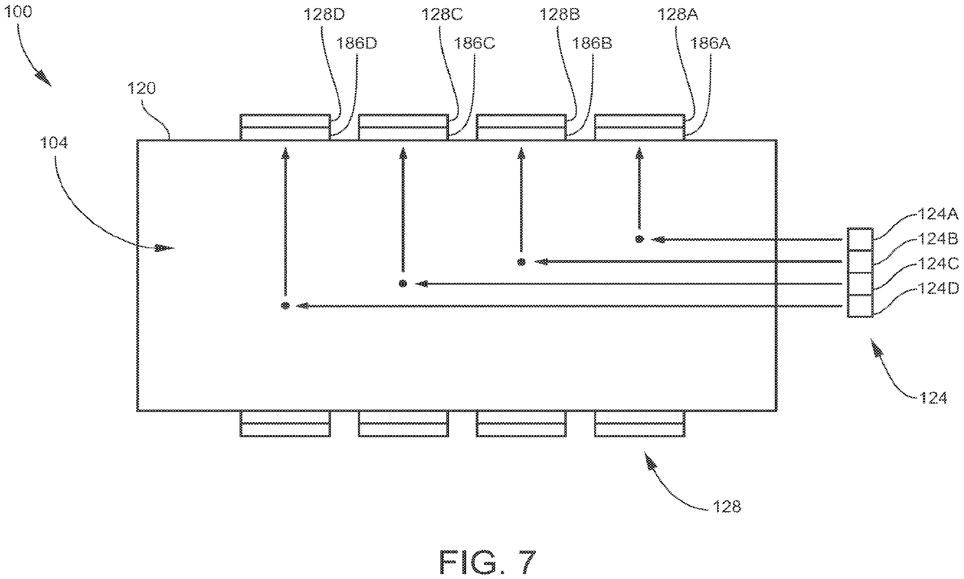

Figure 7 is a schematic cross-sectional view of the particle detector 100 according to other embodiments. The light source 124 may include a plurality of separate light sources (or light source units) 124A, 124B, 124C, and 124D. Four light sources 124A, 124B, 124C, and 124D are illustrated by example only, as more than or less than four may be provided. In some embodiments, the light sources 124A, 124B, 124C, and 124D may be arranged in a closely grouped bundle centered on the longitudinal axis 132. In some embodiments, two or more of the light sources 124A, 124B, 124C, and 124D may emit irradiating light at the same wavelength, which may be useful for boosting the measurement signal and/or increasing the overall size of the particle irradiation region within the detection cavity 104. In some embodiments, at least one of the light sources 124A, 124B, 124C, and 124D may emit irradiating light at a wavelength different from that of the other light sources 124A, 124B, 124C, and 124D. For example, one or more light sources 124A, 124B, 124C, and 124D may emit irradiating light at a first wavelength selected for measuring scattered radiation, while one or more other light sources 124A, 124B, 124C, and 124D may emit irradiating light at a second, different wavelength selected for measuring scattered radiation. As another example, one or more light sources 124A, 124B, 124C, and 124D may emit irradiating light at a first wavelength selected for measuring fluorescent radiation from a first type of particle, while one or more other light sources 124A, 124B, 124C, and 124D may emit irradiating light at a second, different wavelength selected for measuring fluorescent radiation from a second type of particle. The latter configuration may be useful, for example, for detecting one or more types of particles in the sample fluid. As another example, one or more light sources 124A, 124B, 124C, and 124D may emit irradiating light at a first wavelength (or two or more different first wavelengths) selected for measuring scattered radiation, while one or more other light sources 124A, 124B, 124C, and 124D may emit irradiating light at a second, different wavelength (or two or more different second wavelengths) selected for measuring fluorescent radiation.

In some embodiments entailing the use of two or more different irradiation wavelengths, the different light sources 124A, 124B, 124C, and 124D may be operated sequentially according to any desired pulse sequence. For example, the particle detector 100 may alternate the operation of two different light sources 124A, 124B, 124C, and 124D one or more times to alternately measure scattered radiation and fluorescent radiation. As another example, the particle detector 100 may cycle through the operation of two or more different light sources 124A, 124B, 124C, and 124D one or more times to measure scattered radiation at two or more different wavelengths and/or measure fluorescent radiation at two or more different wavelengths.

As also illustrated in Figure 7, the light detector 128 may include a plurality of separate light detectors (or light detector units) 128A, 128B, 124C, and 128D. Each light detector 128A, 128B, 124C, and 128D includes a photoelectric material, which may comprise a plurality of photoelectric units as described above and illustrated in Figure 6, and associated components. Four light detectors 128A, 128B, 124C, and 128D are illustrated by example only, as more than or less than four may be provided. The number of light detectors 128A, 128B, 124C, and 128D may be the same as, less than, or more than the number of light sources 124A, 124B, 124C, and 124D. One or more of the light detectors 128A, 128B, 124C, and 128D may be optically aligned with respective optical filters 186A, 186B, 186C, and 186D.

Providing two or more light detectors 128A, 128B, 124C, and 128D may be done to increase the active detection area of the light detector 128, and to increase the number and angular range of forward-angle paths 344 and back-angle paths 346 (Figure 3) in the line of sight of the active detection area. Alternatively or additionally, providing two or more light detectors 128A, 128B, 124C, and 128D may be done to increase detector signal intensity. In some embodiments, two or more light detectors 128A, 128B, 124C, and 128D may be electrically coupled in series with each other to increase voltage response, and/or two or more light detectors 128A, 128B, 124C, and 128D may be electrically coupled in parallel with each other to increase current response.

Alternatively or additionally, providing two or more light detectors 128A, 128B, 124C, and 128D may be done to provide two or more distinct wavelength (or distinct wavelength range) collection abilities and/or to produce two or more distinct detector output signals. In such embodiments, two or more light detectors 128A, 128B, 124C, and 128D may be electrically isolated from each other, and thus operate independently from each other. For example, this may be done so that the same particle detector 100 may be utilized for both scattering- and fluorescence-based analyses, and/or for scattering-based analyses implemented at two or more different irradiation wavelengths, and/or for fluorescence-based analyses implemented at two or more different irradiation (excitation) wavelengths or two or more different measurement wavelengths (or wavelength ranges). Thus in some embodiments, at least one of the light detectors 128A, 128B, 124C, and 128D may be sensitive to a wavelength (or range of wavelengths) different from the other light detectors 128A, 128B, 124C, and 128D. Alternatively, at least one of the light detectors 128A, 128B, 124C, and 128D may be optically aligned with an optical filter 186A, 186B, 186C that passes to that light detector a wavelength (or range of wavelengths) different from the wavelengths received by the other light detectors 128A, 128B, 124C, and 128D. In one specific example, a light detector intended to receive scattered radiation may include an optical filter that blocks other wavelengths associated with fluorescent radiation, while another light detector intended to receive fluorescent radiation include an optical filter that blocks the wavelength associated with the irradiating light (and thus scattered radiation). In the illustrated example, light detector 128A is configured to collect measurement light scattered or emitted from particles irradiated by light source 124A, light detector 128B is configured to collect measurement light scattered or emitted from particles irradiated by light source 124B, light detector 128C is configured to collect measurement light scattered or emitted from particles irradiated by light source 124C, and light detector 128D is configured to collect measurement light scattered or emitted from particles irradiated by light source 124D.

Generally, the particle properties, attributes of the irradiating light, and signal response of the light detector with respect to wavelength may all influence the LOD and sensitivity of the particle detector 100. Detection of particle type, sensitivity, and LOD may all be tuned (optimized) via appropriate selection of optical filter(s), the wavelength, intensity and collimation of the irradiating light, and the response characteristics of the light detector(s).

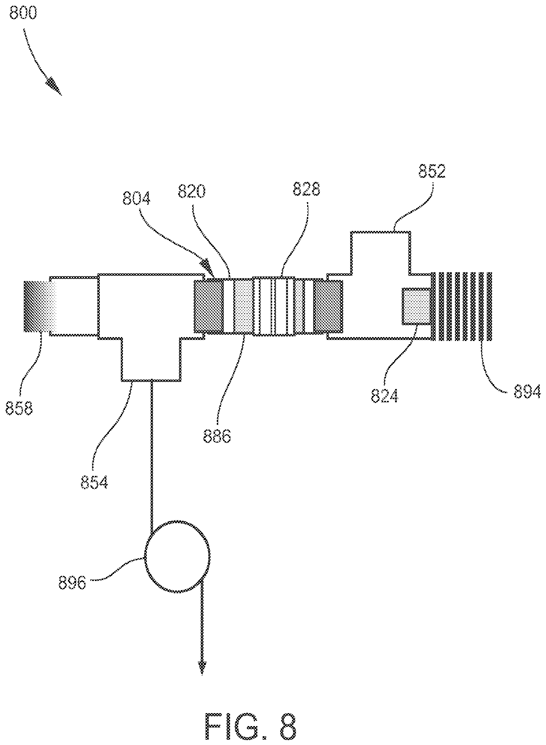

Figure 8 is a schematic plan view of another example of a particle detector 800 according to some embodiments. The particle detector 800 may include a housing 820 defining a flow-through detection cavity 804, one or more light sources 824, and one or more light detectors 828. In the illustrated embodiment, the housing 820 and thus the detection cavity 804 are generally cylindrical and elongated along the longitudinal axis. The particle detector 800 may also include a light trap 858 and one or more optical filters 886 as described above, as well as one or more other features described above and illustrated in Figures 1 to 7. In some embodiments, the light detector(s) 828 and optical filter(s) 886 may include flexible materials as described above. In the present embodiment, the light source 824 includes a heat sink 894, which may provide cooling fins or other means for increasing surface area available for heat transfer with ambient air. The housing 820 includes a sample inlet 852 and a sample outlet 854 positioned such that the housing 820 defines a sample flow path from the sample inlet 852, through the detection cavity 804, and to the sample outlet 854. In the present embodiment, the housing 820 is configured such that the sample inlet 852, the sample outlet 854, or both (as illustrated) are oriented at an angle to the longitudinal axis (ninety degrees in the illustrated example). This configuration minimizes the amount of ambient light entering the detection cavity 804 and reaching the light detector(s) 828.

As also shown in Figure 8, the particle detector 800 may include a fluid moving device 896 (e.g., pump, fan, blower, etc.) configured for moving sample fluid through the sample flow path. Generally, the fluid moving device 896 communicates with the detection cavity 804. For this purpose, the fluid moving device 896 may be positioned downstream of the detection cavity 804. The fluid moving device 896 may be positioned downstream of the sample outlet 854 and outside the housing 820 (as illustrated), or may be positioned inside the housing 820. Generally, the fluid moving device 896 should be positioned so as not to create turbulence in the detection cavity 804. The fluid moving device 896 may be configured for moving the sample fluid through the detection cavity 804 under laminar flow conditions and in a smooth, pulse-free manner. Maintaining the laminar flow regime may minimize internal particle losses and improve the sensitivity and accuracy of the data acquired. In some embodiments, the fluid moving device 896 is configured for moving the sample fluid through the detection cavity 804 at a flow rate on the order of liters per minute. In some embodiments, the fluid moving device 896 is configured such that the flow rate is adjustable by a user. It will be understood that the fluid moving device 896 is optional. Ambient fluid flow conditions may be sufficient for operating the particle detector 800 without the use of the fluid moving device 896.

As also shown in Figure 8, the particle detector 800 may have a modular configuration in which one or more housing portions and/or components of the particle detector 800 may be removable from other housing portions or components (e.g., light source(s) 824, light trap 858, fluid moving device 896) for cleaning, maintenance, or replacement. Light source(s) 824 may also be removable to enable a user to select different irradiation wavelengths. Light detector(s) 828 and optical filter(s) 886, or the housing portion on which the light detector(s) 828 and optical filter(s) 886 are attached, may be removable to enable a user to select different light detectors 828 and optical filters 886 or combinations thereof. Moreover, the modularity may enable the user to add different housing portions in series to configure the particle detector 800 in a manner similar to that shown in Figure 7. The multiple housing portions may include the same or different combinations of one or more light detectors 828 and one or more optical filters 886 pre-attached to the housing portions.

The particle detector 800 may be provided to the user in the form of a kit in which the particle detector 800 is fully or partially disassembled. For example, the kit may include a plurality of different light sources 824, light detectors 828, and/or optical filters 886. Alternatively or additionally, the kit may include a plurality of different housing portions defining the detection cavity 804. The housing portions may include different combinations of one or more light detectors 828 and one or more optical filters 886 pre-attached to the housing portions, thereby enabling the user to tailor the analytical functions of the particle detector 800 as desired.

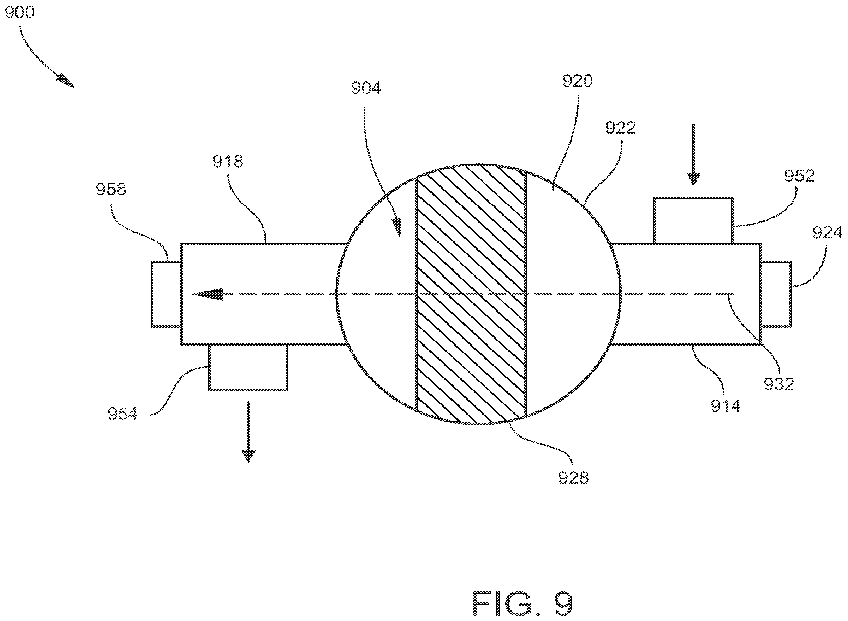

Figure 9 is a schematic plan view of another example of a particle detector 900. The particle detector 900 may include a housing 920 defining a flow-through detection cavity 904, one or more light sources 924, and one or more light detectors 928. The particle detector 900 may also include a light trap 958 and one or more optical filters (not shown) as described above, as well as one or more other features described above and illustrated in Figures 1 to 8. In some embodiments, the light detector(s) 928 and optical filter(s) may include flexible materials as described above. The housing 920 includes a sample inlet 952 and a sample outlet 954 positioned such that the housing 920 defines a sample flow path from the sample inlet 952, through the detection cavity 904, and to the sample outlet 954. The light source 924, sample inlet 952, sample outlet 954, and light trap 958 may be positioned such that the irradiating light propagates and sample fluid flows generally collinearly along a longitudinal axis 932. The particle detector 900 may be configured similarly to the particle detector 800 described above and illustrated in Figure 8. However, the particle detector 900 includes one or more sections in which the size of the cross-section (cross-sectional area) of the detection cavity 904 varies along the longitudinal axis 932. In the illustrated embodiment, this is implemented by the housing 920 and thus the detection cavity 904 being spherical or including a spherical section 922. In this case, the longitudinal axis 932 may be an axis of symmetry of the spherical section 922.

The housing 920 may include an axial inlet section 914 and an axial outlet section 918 of rounded or polygonal cross-section, extending from the spherical section 922 along the longitudinal axis 932. As illustrated, the light source 924 and sample inlet 952 may be positioned at the inlet section 914 and the sample outlet 954 and the light trap 958 may be positioned at the outlet section 918. The light detector 928 may be wrapped around the detection cavity 904 in an orientation ninety degrees to the longitudinal axis 932, or may be oriented at a different angle. Additional light detectors (not shown) may be wrapped fully or partially around the detection cavity 904 to provide additional areas for active detection.

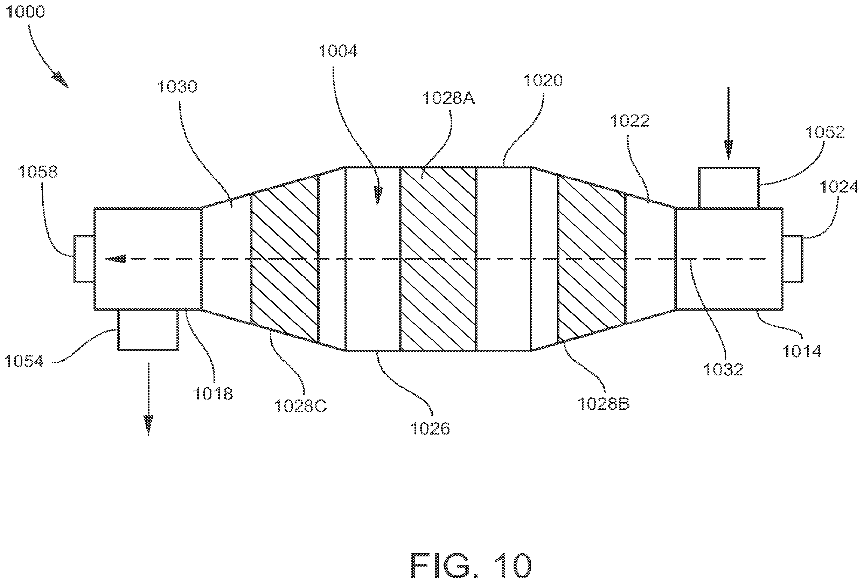

Figure 10 is a schematic plan view of another example of a particle detector 1000 according to some embodiments. The particle detector 1000 may include a housing 1020 defining a flow-through detection cavity 1004, one or more light sources 1024, and one or more light detectors 1028A, 1028B, and 1028C. The particle detector 1000 may also include a light trap 1058 and one or more optical filters (not shown) as described above, as well as one or more other features described above and illustrated in Figures 1 to 9. The light detector(s) 1028A, 1028B, and 1028C and optical filter(s) includes flexible materials as described above. The cross-sections of the housing 1020 and detection cavity 1004 may be rounded or polygonal. The housing 1020 includes a sample inlet 1052 and a sample outlet 1054 positioned such that the housing 1020 defines a sample flow path from the sample inlet 1052, through the detection cavity 1004, and to the sample outlet 1054. The light source 1024, sample inlet 1052, sample outlet 1054, and light trap 1058 may be positioned such that the irradiating light propagates and sample fluid flows generally collinearly along a longitudinal axis 1032, which may be an axis of symmetry of the detection cavity 1004 and one or more other portions of the particle detector 1000. The particle detector 1000 may be configured similarly to the particle detector 800 described above and illustrated in Figure 8 or 9. However, the particle detector 1000 includes one or more sections in which the size of the cross-section (cross-sectional area) of the detection cavity 1004 varies along the longitudinal axis 1032. This may be implemented, for example, by the housing 1020 including one or more transitions, or tapered sections, in which the cross-section increases or decreases. Such transitions or tapered sections may have, for example, a truncated conical or pyramidal configuration. In the illustrated embodiment, the housing 1020 includes an increasing transition 1022 (i.e., the cross-section increases in the direction of fluid flow and irradiating light propagation) adjoined to a section 1026 of constant cross-section, which in turn is adjoined to a decreasing transition 1030.

In another embodiment, the transition 1022 may be a decreasing transition instead of an increasing transition, whereby the cross-section decreases in the direction of fluid flow such that the flow is focused into a smaller cross-section in the section 1026 of constant cross-section. More generally, the determination as to whether to include cross-sectional transitions, and whether such transitions should expand or converge the cross-section in the direction of fluid flow, may depend on a variety of factors related to fluid mechanics, distance between particles and particle detector, etc.

Also in the illustrated embodiment, the housing 1020 may include an axial inlet section 1014 extending from the increasing transition 1022, and an axial outlet section 1018 extending from the decreasing transition 1030 along the longitudinal axis 1032. As illustrated, the light source 1024 and sample inlet 1052 may be positioned at the inlet section 1014 and the sample outlet 1054 and the light trap 1058 may be positioned at the outlet section 1018. One or more light detectors 1028A may be wrapped around the detection cavity 1004 at the section 1026 of constant cross-section, in an orientation ninety degrees to the longitudinal axis 1032. Additionally or alternatively, one or more light detectors 1028B and/or 1028C may be wrapped around the increasing transition 1022 and/or the decreasing transition 1030, respectively.

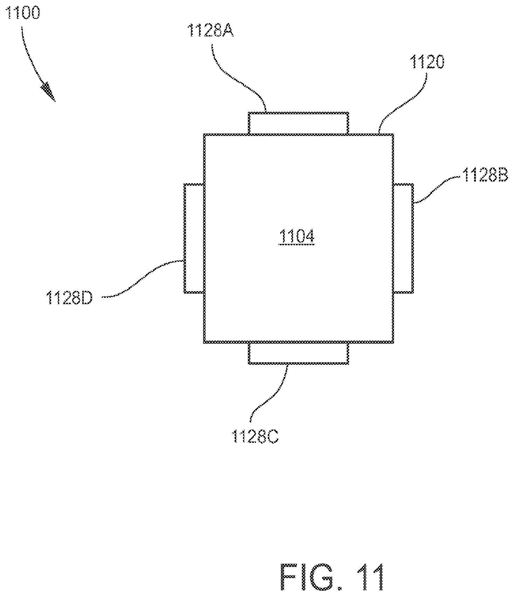

Figure 11 is a schematic cross-sectional view of another example of a particle detector 1100, not forming part of the present invention. The view is taken through a detection cavity 1104 defined by a housing 1120 of the particle detector 1100. The housing 1120 includes a plurality of flat wall sections whereby the cross-section of the detection cavity 1104 is polygonal. Figure 11 illustrates a rectilinear cross-section by example only, as other polygonal geometries (e.g., hexagon, octagon, etc.) may be implemented as well. One or more light detectors 1128A, 1128B, 1128C, and 1128D may be positioned at respective flat wall sections. A lesser number of opposing pairs of light detectors may be provided for a given polygonal geometry. In Figure 11, for example, just one opposing pair of light detectors (1128A and 1128C, or 1128B and 1128D) may be provided.

Particle detectors such as described herein may provide one or more advantages. The particle detectors, particularly with the light detectors such as described herein, may provide a simple, low-cost solution to measuring particle concentration, and have been demonstrated through testing to be very sensitive, yielding high photon collection without requiring precision beam shaping optics (e.g., lenses and mirrors) in the measurement light path. That is, the light detector may enable the detection cavity to be free of beam shaping optics in the measurement light paths between the longitudinal axis and the photoelectric material. This is due at least in part to the light detector having a large-area active photoelectric material that conformally surrounds the detection cavity, whereby the light detector is able to receive measurement light over nearly all directions of propagation. Moreover, with the active detection area being large and conformally surrounding the detection cavity, the light detector may enable the detection cavity to have a much larger volume compared to conventional devices, allowing for a significantly larger fraction and number of photons of scattered or fluorescent radiation to be collected during the transit time of the sample fluid past the light detector, and longer transit times (e.g., on the order of seconds or tenths of seconds for liter/min flow rates such as 5 L/min). This is in contrast to the light detectors utilized in conventional particle detectors, which are able to capture only a small fraction of photons over transit times of about 1 to 10 microseconds (µs). It will be noted that although the flow rate × transient time = constant; characteristics of the irradiation source, photoelectric material, and measurement electronics may result in there being an optimum flow rate for sensitivity and/or LOD of the particle detector. The configuration and detection methodology of the light detector may also significantly relax requirements for accuracy and precision in the alignment of the light source in relation to the detection cavity or other components of the particle detector, as compared to conventional single particle counters with small detection cavities and multiple beam shaping optics. Light detectors such as described herein also enable measurement of the total concentration of particles in a volume of sample fluid (#/cm³) and changes in concentration, as opposed to conventional single particle counting techniques. This approach may simplify the optics required and eliminates the need for focusing the sample fluid into a single particle flow path. In addition, the simple geometry of the detection cavity (e.g., cylindrical) may simplify the assembly and maintenance of the particle detector, minimize deposition of particles on internal surfaces, and make cleaning easier.

### EXAMPLE 1 - General Aerosol Detection

In this Example, a particle detector having a configuration similar to that illustrated in Figure 8 was fabricated. The housing was an optically clear tube that was 1.62 inches (41.1 mm) in diameter. A flexible PV detector sized at 1.5 inches by 4.5 inches (MP3-37 by Power Film Solar) was selected as the light detector. The flexible PV detector was wrapped around the clear tube. A red laser diode of 650 nm wavelength was selected as the light source, and positioned to shine down the center of the tube with air flowing coaxial with the light at a flow rate of 5 liters per minute. A light trap was positioned at the opposite end of the tube. A test system was constructed to evaluate the particle detector. The test system was made up of mostly black polyvinylchloride (PVC) pipe that was 1½ inches in diameter. The output voltage of the PV detector was measured in real time at 1 sample per second using a 16 Bit DataLogger (Measurement Computing USB-1698FS-Plus).

The particle detector was connected to an aerosol mixing chamber that supplied a controlled and well mixed concentration of aerosol. An Aerodynamic Particle Sizer (APS; TSI Incorporated) was placed after (downstream of) the particle detector. The APS provided the air flow (5 L/min) through the particle detector and provided size and count information about the aerosol passing through the instruments. This arrangement of the sampled air flowing through both instruments provided identical aerosol concentrations to both instruments. The aerosol concentration information from the APS was used as a standard or reference. In addition to the APS, a Condensation Particle Counter model 3022a (CPC; TSI Incorporated) was also connected to the aerosol mixing chamber but not in the same sampling flow path as the particle detector and APS. However, given the well mixed environment of the aerosol chamber, very similar concentrations were expected to be measured by all instruments. Using the combination of the two particle counters provided better insight into how well the particle detector performed. The APS measures particle size and concentration ranging from approximately 0.5 µm (500 nm) to over 5 µm. The CPC model 3022a has a lower size range of 7 nm, and an upper range of approximately 1 µm. Used by itself the CPC provides only total count information. Adding a Scanning Mobility Particle Sizer (SMPS; TSI Incorporated) provided size information. Both of the APS and CPC instruments are precision particle counters.

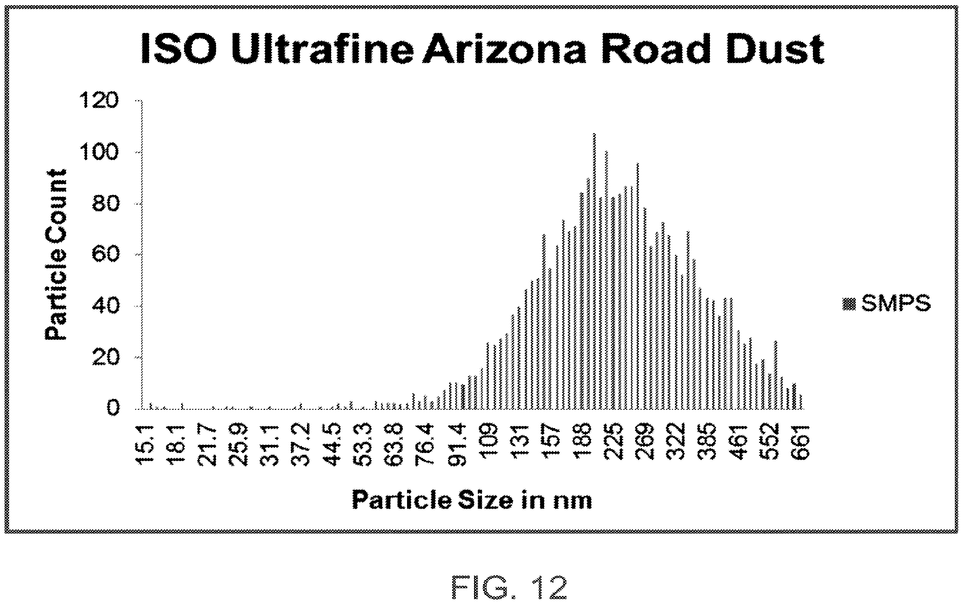

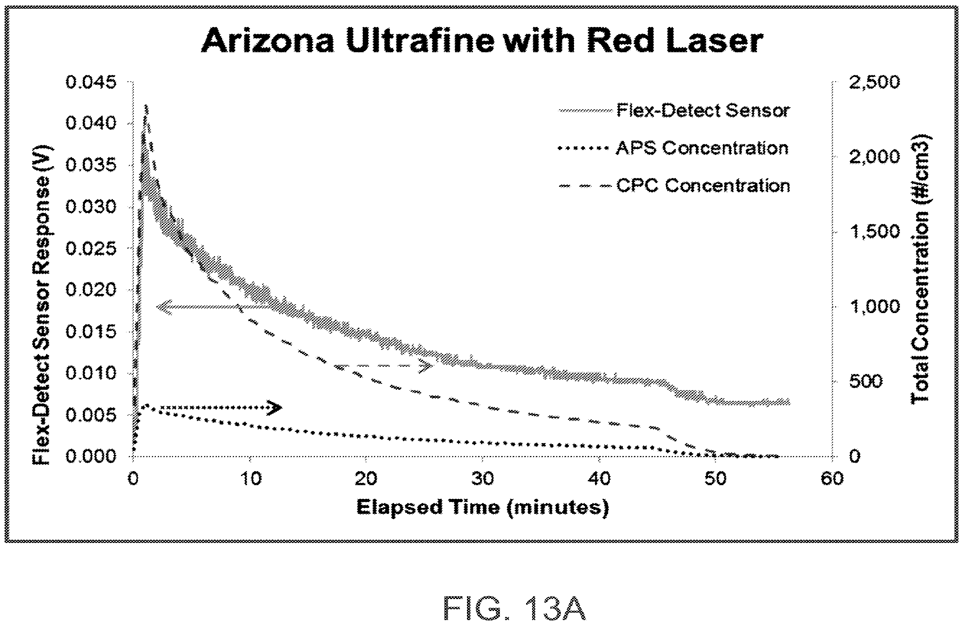

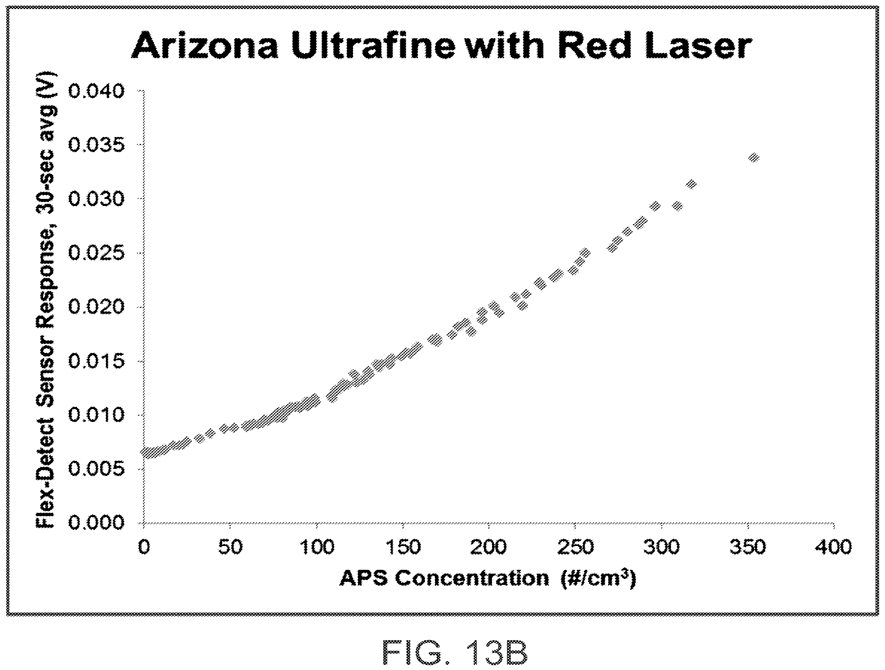

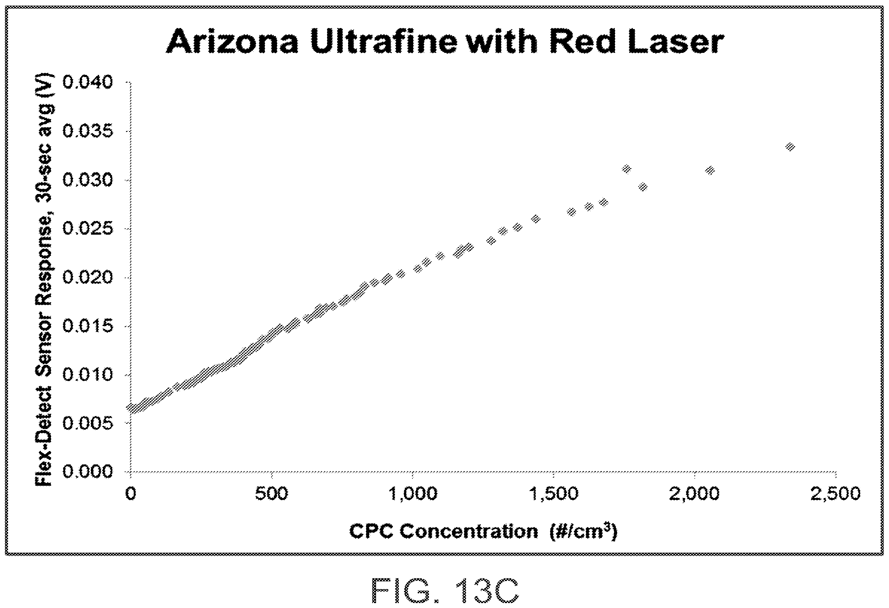

Experiments were conducted using ISO 12103-1, A1 Ultrafine Test Dust (also called Arizona Road Dust). The median size range of the test dust was around 250 nm as measured by the SMPS and shown in Figure 12. Size distribution of ISO Ultrafine Arizona Road Dust as measured with a TSI SMPS. A typical experiment involved dispersing a small amount of Arizona Road Dust into the aerosol mixing chamber using a TSI Model 3433 Small Scale Powder Disperser. The well mixed aerosol in the chamber was then sampled by the particle detector and the two commercial particle counters (APS and CPC). The aerosol concentration in the chamber slowly decayed over time until the point when a clean out pump was turned on, which drew the aerosol out of the chamber while introducing filtered air into the test chamber. This decay in concentration followed by the clean out is shown in Figure 13A. The particle detector showed response to the change in concentration similar to the two commercial particle counters. A direct comparison of the particle detector and the two commercial particle counters is shown in Figures 13B and 13C. It is noted that with the configuration of the particle detector employed in this experiment, the particle detector provided more of a mass concentration measurement and did not account for particle size. This difference was reflected in the lack of a true 1:1 relationship of the particle detector response and that of the two commercial particle counters. Using different wavelengths of light and calibration algorithms the accuracy of the measurement could be improved. Using a 650 nm red laser in the particle detector, concentrations below 0.9 particles/cm³ and sizes as small as at least 0.25 µm (25 nm) were detected by the particle detector.

### EXAMPLE 2 - Bioaerosol Detection

In this Example, a particle detector having a configuration similar to that described above in Example 1 was fabricated. However, a 365 nm UV LED with a collimating lens was utilized as the excitation source, and an aluminum heat sink was utilized to hold the LED and maintain a stable temperature. The excitation wavelength was blocked by using a Rosco 400 nm UV filter gel sheet. The UV filter was wrapped around the clear tube. The flexible PV detector was then wrapped around the gel filter thus creating a detection cavity that excludes the excitation radiation from the PV detector.

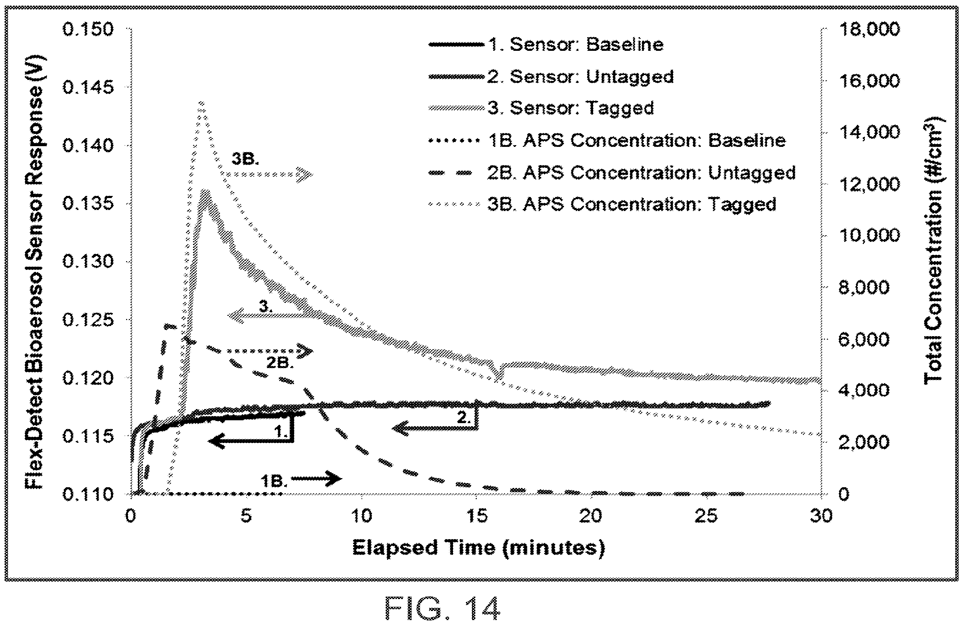

Using the same experimental set up as described above in Example 1, a fluorescent aerosol was used as a simulant for a bioaerosol. WR Grace Syloid powder was tagged with 2% Tinopal CBS X. The Tinopal tag has an absorbance maximum of approximately 385 nm. The emission wavelength maximum occurs at approximately 430 nm. Control experiments were conducted using untagged Syloid aerosol to demonstrate that the signal detected by the PV detector was not scattered excitation energy but rather emitted photons from fluorescence. Figure 14 compares the response of the particle detector to tagged and untagged Syloid aerosol. In this set of experiments stray source radiation was reaching the PV detector, thereby limiting the level of detection.

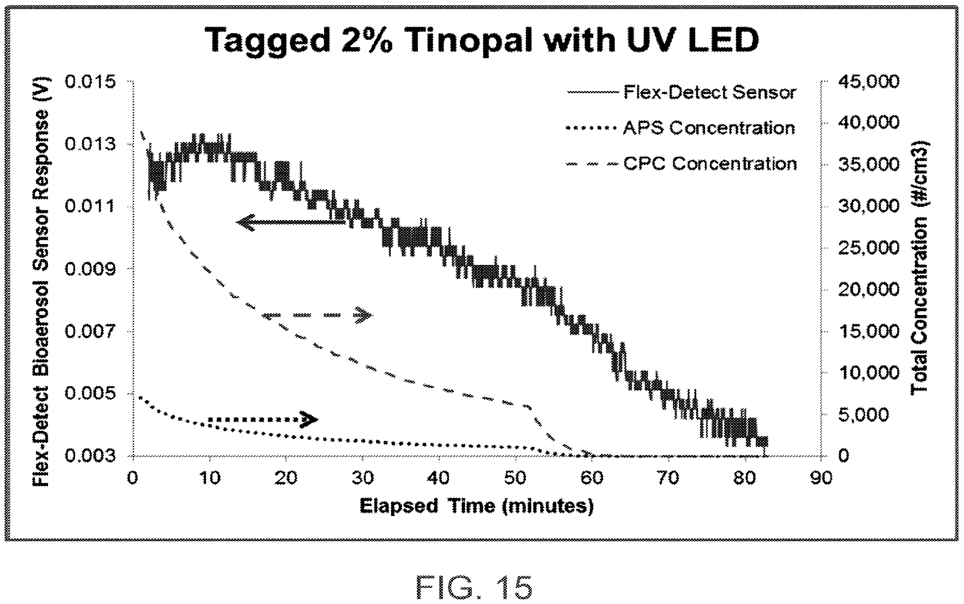

Adding apertures and improving the collimation of the LED, a second set of experiments was carried out that provided both a lower limit of detection and more sensitivity as shown in Figure 15. Further improvements are possible with additional improvements to reducing stray source radiation and optimization of the geometry of the sensing cavity and sample flow rate.

### EXAMPLE 3 - Bioaerosol Detection

In this Example, a particle detector having a configuration similar to that described above in Example 2 was fabricated. However, the particle detector was reconfigured to use a 405 nm violet laser. A similar experimental setup was used as described above in Example 2, except that a controlled concentration of bioaerosol from *Bacillus* spores was introduced into the chamber and sampled with the two commercial particle counters described above and the particle detector. The 405 nm wavelength is at the upper end of excitation of bioparticles. However, a laser provides an intense, coherent light effectively delivered to the bioaerosol in the detection cavity. Violet lasers of 405 nm have previously been demonstrated in detecting bioaerosols. *See* Saari et al., "Performance of Two Fluorescence-Based Real-Time Bioaerosol Detectors: BioScout vs. UVAPS," Aerosol Science and Technology 48(4): 371-378 (2014). The 405 nm laser both scatters elastically (total aerosol detection) and stimulates fluoresce (bioaerosol detection). However, the gel filter used in front of the PV detector in this experiment only blocked excitation light below 400 nm, so both scattered and emitted fluorescence were detected. A second setup was used with just a 650 nm red laser to measure only total aerosol concentration (no fluorescence stimulated). This difference of the signals from the 405 nm and 650 nm provided a crude estimation of the fluorescence signal. Ideally a filter that excludes the 405 excitation light should be employed. Alternatively, UV LEDs with wavelengths below 400 nm could be employed but careful collimation of the light is required.

A simulant for anthrax bacteria, *Bacillus atrophaeus* (*Bg*), was used to test the particle detector. A known concentration of *Bg* spores was injected into the aerosol test chamber using a 3 jet collision nebulizer. The particle detector and APS took data from before injection of *Bg* spores until an unmeasurable concentration was achieved. Viable sampling was done via an all-glass impinger, 4 mm (AGI-4) using impinger fluid to collect the spores. Plating and counting of colonies was used to determine viable counts.

The AGI results were combined with the APS particle size information to estimate time resolved viable bioaerosol concentration. A distinct peak formed around 1 µm when the *Bg* spores were being injected. Other particles present during this event could be growth media or extracellular material. A view of the particle distribution by mass concentration also revealed the tight distribution of single spores. With that information, it is reasonable to extract the size bins of interest from the particle data. In this case, the log distributed average bin sizes of 0.965 µm, 1.037 µm, and 1.114 µm were selected to compensate for binning inconsistencies. These three bins were summed to provide time-resolved particle number concentration information. However, the bracketing bins may also contain some non-spore material, thus providing an overestimate of total spore concentration.

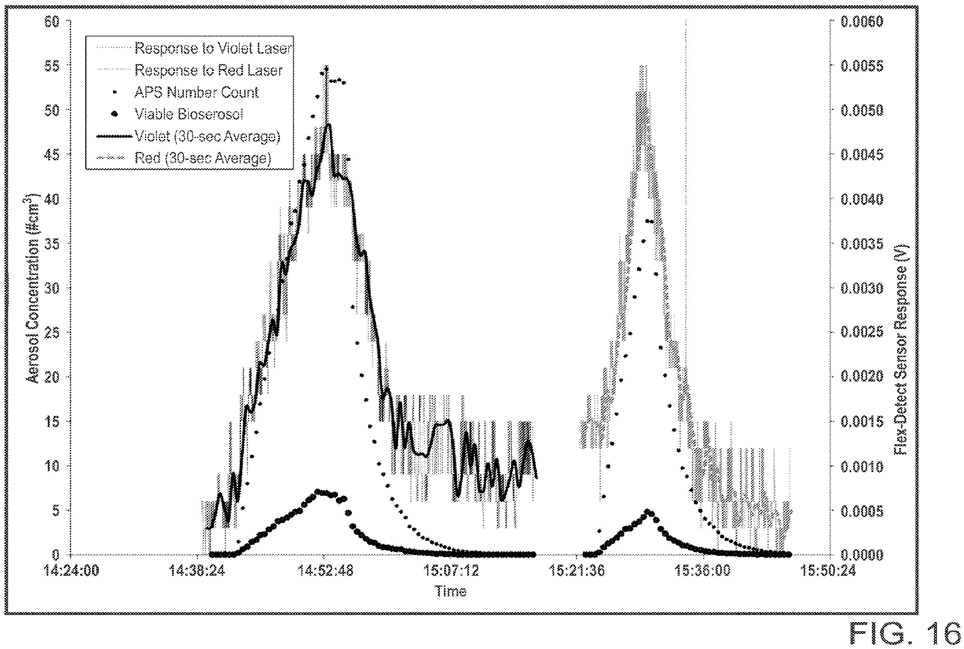

Figure 16 shows the results for the particle detector for the *Bg* bioaerosol challenge for measurements with the 405 nm and 650 nm lasers, compared to the APS and estimated viable bioaerosol concentration. The particle detector tracked nicely with the APS concentration, detecting the change in *Bg* concentration from time of injection to decay after the injection was stopped. The lower limit measured by the particle detector was on the order of 0.085 #/cm³.

In general, terms such as "communicate" and "in ... communication with" (for example, a first component "communicates with" or "is in communication with" a second component) are used herein to indicate a structural, functional, mechanical, electrical, signal, optical, magnetic, electromagnetic, ionic or fluidic relationship between two or more components or elements. As such, the fact that one component is said to communicate with a second component is not intended to exclude the possibility that additional components may be present between, and/or operatively associated or engaged with, the first and second components.

It will be understood that the foregoing description is for the purpose of illustration only, and not for the purpose of limitation-the invention being defined by the claims.

## Claims

1. A particle detector (100, 800, 900, 1000, 1100), comprising:
a housing (120, 820, 920, 1020, 1120) comprising a sample inlet (152, 852, 952, 1052) and a sample outlet (154, 854, 954, 1054), and enclosing a detection cavity (104, 804, 904, 1004, 1104) having a cavity length along a longitudinal axis (132, 932, 1032), wherein the housing defines a flow path for a sample fluid from the sample inlet (152, 852, 952, 1052), through the detection cavity (104, 804, 904, 1004, 1104), and to the sample outlet (154, 854, 954, 1054); and
a light source (124, 824, 924, 1024) configured for directing irradiating light along the longitudinal axis (132, 932, 1032) to particles (112, 136) of the sample fluid flowing in the detection cavity (104, 804, 904, 1004, 1104);
a flexible photoelectric material (678) conformally disposed on the housing (120, 820, 920, 1020, 1120) and surrounding the detection cavity (104, 804, 904, 1004, 1104) such that, along at least a portion of the cavity length, the photoelectric material (678) extends around the longitudinal axis (132, 932, 1032) through an arc length in a range from 30° to 360° in a plane orthogonal to the longitudinal axis,
wherein the photoelectric material (678) is configured for receiving measurement light propagating from the particles (112, 136) in a plurality of measurement light paths (116, 342, 344, 346) angled relative to the longitudinal axis (132, 932, 1032).

2. The particle detector of claim 1, wherein the detection cavity (104, 804, 904, 1004, 1104) has a configuration selected from the group consisting of:
the detection cavity is generally cylindrical, spherical, or polygonal;
at least a portion of the detection cavity has a cross-sectional area that varies along the longitudinal axis (132, 932, 1032);
the detection cavity comprises a transition (1022) having a cross-sectional area that increases along the longitudinal axis (132, 932, 1032);
the detection cavity comprises a transition (1030) having a cross-sectional area that decreases along the longitudinal axis (132, 932, 1032);
the detection cavity is free of beam shaping optics in the measurement light paths (116, 342, 344, 346) between the longitudinal axis (132, 932, 1032) and the photoelectric material (678);
the detection cavity comprises a first end and an opposing second end on the longitudinal axis (132, 932, 1032), and the light source (124, 824, 924, 1024) is configured for directing the irradiating light from the first end toward the second end;
the detection cavity comprises a first end and an opposing second end on the longitudinal axis (132, 932, 1032), and the light source (124, 824, 924, 1024) is mounted to the housing (120, 820, 920, 1020, 1120) at the first end; and
a combination of two or more of the foregoing.

3. The particle detector of claim 1 or 2, wherein the light source (124, 824, 924, 1024) has a configuration selected from the group consisting of:
the light source is configured for emitting the irradiating light as a coherent beam, a collimated beam, or both a coherent and collimated beam;
the light source is configured for emitting the irradiating light having a beam diameter in a range from 0.4 mm to 4000 mm;
the light source is configured for emitting the irradiating light having a cross-sectional area in a range from 1% to 80% of a cross-sectional area of the detection cavity (104, 804, 904, 1004, 1104);
the light source is configured for emitting the irradiating light at an irradiating wavelength in the ultraviolet range, in the visible range, or in the infrared range;
the light source is configured for emitting the irradiating light at an irradiating wavelength in a range from 250 to 1500 nm;
the light source is configured for emitting the irradiating light at an irradiating wavelength in a range effective for inducing autofluorescence in one or more types of bio-particles;
a combination of two or more of the foregoing.

4. The particle detector of any of the preceding claims, wherein the light source (124, 824, 924, 1024) comprises a plurality of light sources, wherein at least one of the light sources is configured for emitting the irradiating light at an irradiating wavelength different from the other light sources.

5. The particle detector of any of the preceding claims, comprising an optical filter (186, 886) positioned between the photoelectric material (678) and the longitudinal axis (132, 932, 1032), wherein the measurement light passes through the optical filter.

6. The particle detector of claim 5, wherein the light source (124, 824, 924, 1024) is configured for emitting the irradiating light at an irradiating wavelength, and the optical filter (186, 886) is configured for blocking undesired photons from impinging on the photoelectric material (678), the undesired photons having a wavelength selected from the group consisting of:
wavelengths in or more wavelength ranges other than the irradiating wavelength; wavelengths in or more wavelength ranges other than a wavelength range in which one or more types of bio-particles fluoresce; and
wavelengths in or more wavelength ranges other than the irradiating wavelength, and other than a wavelength range in which one or more types of bio-particles fluoresce.

7. The particle detector of claim 5 or 6, wherein the housing (120, 820, 920, 1020, 1120) comprises an outside surface and an inside surface, and the photoelectric material (678) and the optical filter (186, 886) are positioned according to an arrangement selected from the group consisting of:
the photoelectric material is conformally disposed on the outside surface, and the optical filter is conformally disposed on the inside surface;
the optical filter is conformally disposed on the outside surface, and the photoelectric material is conformally disposed on the optical filter; and
the photoelectric material is conformally disposed on the inside surface, and the optical filter is conformally disposed on the photoelectric material.

8. The particle detector of any of the preceding claims, wherein the photoelectric material (678) is a photovoltaic material.

9. The particle detector of any of the preceding claims, wherein the photoelectric material (678) comprises a plurality of photoelectric units (682) positioned in proximity to each other, wherein in particular the photoelectric units (682) are arranged at different angular positions relative to the longitudinal axis (132, 932, 1032), at different axial positions relative to the longitudinal axis, or both at different angular positions and axial positions relative to the longitudinal axis.

10. The particle detector of any of the preceding claims, wherein the photoelectric material (678) comprises a plurality of photoelectric materials, wherein in particular the plurality of photoelectric materials (678) has a configuration selected from the group consisting of:
the plurality of photoelectric materials comprises two or more photoelectric materials electrically coupled in series with each other;
the plurality of photoelectric materials comprises two or more photoelectric materials electrically coupled in parallel with each other;
the plurality of photoelectric materials comprises two or more photoelectric materials electrically isolated from each other; and
a combination of two or more of the foregoing.

11. The particle detector of claim 10, wherein the plurality of photoelectric materials (682) comprises a plurality of electrically isolated photoelectric materials, and further comprising a plurality of optical filters (186, 886) respectively positioned between one or more of the electrically isolated photoelectric materials and the longitudinal axis (132, 932, 1032) such that the measurement light passes through the optical filters, wherein at least one of the optical filters is configured for passing a wavelength range of the measurement light different from a wavelength range passed by the other optical filters.

12. The particle detector of claim 11, wherein the at least one optical filter (186, 886) is configured for passing a wavelength range corresponding to measurement light fluorescently emitted by the particles (112, 136) while blocking a wavelength range corresponding to measurement light scattered by the particles.

13. A method for measuring particles (112, 136) in a sample fluid, the method comprising:
flowing the sample fluid through a detection cavity (104, 804, 904, 1004, 1104), wherein the detection cavity (104, 804, 904, 1004, 1104) has a cavity length along a longitudinal axis (132, 932, 1032) and wherein the detection cavity (104, 804, 904, 1004, 1104) is enclosed by a housing (120, 820, 920, 1020, 1120);
directing an irradiating light through the detection cavity (104, 804, 904, 1004, 1104) along said longitudinal axis (132, 932, 1032) to irradiate particles in the sample fluid, wherein the particles emit measurement light in response to the irradiation; and
receiving at a photoelectrical material (678) measurement light propagating from the particles (112, 136) in a plurality of measurement light paths (116, 342, 344, 346) angled relative to the longitudinal axis (132),
**characterized in that**
the photoelectric material is flexible and it is conformally disposed on the housing (120, 820, 920, 1020, 1120) and surrounds the detection cavity (104, 804, 904, 1004, 1104) such that, along at least a portion of the cavity length, the photoelectric material (678) extends around the longitudinal axis (132, 932, 1032) through an arc length in a range from 30° to 360° in a plane orthogonal to the longitudinal axis.

## Patentansprüche

1. Ein Teilchendetektor (100, 800, 900, 1000, 1100), der aufweist:
ein Gehäuse (120, 820, 920, 1020, 1120), das einen Probeneinlass (152, 852, 952, 1052) und einen Probenauslass (154, 854, 954, 1054) aufweist, und das einen Detektionshohlraum (104, 804, 904, 1004, 1104) eine Hohlraumlänge entlang einer Längsachse (132, 932, 1032) umschließt, wobei das Gehäuse einen Strömungsweg für eine Probenflüssigkeit von dem Probeneinlass (152, 852, 952, 1052) durch den Detektionshohlraum (104, 804, 904, 1004, 1104) und zu dem Probenauslass (154, 854, 954, 1054), definiert; und
eine Lichtquelle (124, 824, 924, 1024), die so konfiguriert ist, dass sie Bestrahlungslicht entlang der Längsachse (132, 932, 1032) auf Partikel (112, 136) von der Probenflüssigkeit, die in dem Detektionshohlraum (104, 804, 904, 1004, 1104) strömt, richtet;
ein flexibles photoelektrisches Material (678), das konform auf dem Gehäuse (120, 820, 920, 1020, 1120) angeordnet ist und den Detektionshohlraum (104, 804, 904, 1004) umgibt, so dass sich das photoelektrische Material (678) entlang mindestens eines Teils der Hohlraumlänge um die Längsachse (132, 932, 1032) über eine Bogenlänge in einem Bereich von 30° bis 360° in einer Ebene orthogonal zu der Längsachse erstreckt,
wobei das photoelektrische Material (678) so konfiguriert ist, dass es Messlicht empfängt, das sich von den Partikeln (112, 136) in einer Vielzahl von Messlichtpfaden (116, 342, 344, 346), die relativ zu der Längsachse (132, 932, 1032) unter einem Winkel stehen, ausbreitet.

2. Der Teilchendetektor nach Anspruch 1, wobei der Detektionshohlraum (104, 804, 904, 1004, 1104) eine Konfiguration hat, die ausgewählt ist aus der Gruppe bestehend aus:
der Detektionshohlraum ist allgemein zylindrisch, kugelförmig oder polygonal;
mindestens ein Teil des Detektionshohlraums hat eine Querschnittsfläche, die entlang der Längsachse (132, 932, 1032) variiert;
der Detektionshohlraum weist einen Übergang (1022) mit einer Querschnittsfläche auf, die entlang der Längsachse (132, 932, 1032) zunimmt;
der Detektionshohlraum weist einen Übergang (1030) mit einer Querschnittsfläche auf, die sich entlang der Längsachse (132, 932, 1032) verkleinert;
der Detektionshohlraum ist frei von Strahlformungsoptiken in den Messlichtpfaden (116, 342, 344, 346) zwischen der Längsachse (132, 932, 1032) und dem photoelektrischen Material (678);
der Detektionshohlraum weist ein erstes Ende und ein gegenüberliegendes zweites Ende auf der Längsachse (132, 932, 1032) auf, und die Lichtquelle (124, 824, 924, 1024) ist so konfiguriert, dass sie das Bestrahlungslicht von dem ersten Ende in Richtung des zweiten Endes richtet;
der Detektionshohlraum weist ein erstes Ende und ein gegenüberliegendes zweites Ende auf der Längsachse (132, 932, 1032) auf, und die Lichtquelle (124, 824, 924, 1024) ist an dem Gehäuse (120, 820, 920, 1020, 1120) an dem ersten Ende befestigt; und
eine Kombination aus zwei oder mehreren der vorgenannten Möglichkeiten.

3. Der Teilchendetektor nach Anspruch 1 oder 2, wobei die Lichtquelle (124, 824, 924, 1024) eine Konfiguration aufweist, die ausgewählt ist aus der Gruppe bestehend aus:
die Lichtquelle ist so konfiguriert, dass sie das Bestrahlungslicht als einen kohärenten Strahl, einen kollimierten Strahl oder sowohl als einen kohärenten als auch kollimierten Strahl emittiert;
die Lichtquelle ist so konfiguriert, dass sie das Bestrahlungslicht mit einem Strahlendurchmesser in einem Bereich von 0,4 mm bis 4000 mm emittiert;
die Lichtquelle ist so konfiguriert, dass sie das Bestrahlungslicht mit einer Querschnittsfläche in einem Bereich von 1 % bis 80 % von einer Querschnittsfläche des Detektionshohlraums (104, 804, 904, 1004, 1104) emittiert;
die Lichtquelle ist so konfiguriert, dass sie das Bestrahlungslicht bei einer Bestrahlungswellenlänge im ultravioletten Bereich, im sichtbaren Bereich oder im infraroten Bereich emittiert;
die Lichtquelle ist so konfiguriert, dass sie das Bestrahlungslicht bei einer Bestrahlungswellenlänge in einem Bereich von 250 bis 1500 nm emittiert;
die Lichtquelle ist so konfiguriert, dass sie das Bestrahlungslicht bei einer Bestrahlungswellenlänge in einem Bereich emittiert, der zum Induzieren von Autofluoreszenz in einem oder mehreren Typen von Bio-Partikeln wirksam ist;
eine Kombination aus zwei oder mehreren der vorgenannten Möglichkeiten.

4. Der Teilchendetektor nach einem der vorhergehenden Ansprüche, wobei die Lichtquelle (124, 824, 924, 1024) eine Vielzahl von Lichtquellen aufweist, wobei mindestens eine der Lichtquellen so konfiguriert ist, dass sie das Bestrahlungslicht bei einer Bestrahlungswellenlänge, die sich von den anderen Lichtquellen unterscheidet, emittiert.

5. Der Teilchendetektor nach einem der vorhergehenden Ansprüche, der einen optischen Filter (186, 886) aufweist, der zwischen dem photoelektrischen Material (678) und der Längsachse (132, 932, 1032) angeordnet ist, wobei das Messlicht durch den optischen Filter hindurchgeht.

6. Der Teilchendetektor nach Anspruch 5, wobei die Lichtquelle (124, 824, 924, 1024) so konfiguriert ist, dass sie das Bestrahlungslicht bei einer Bestrahlungswellenlänge emittiert, und der optische Filter (186, 886) so konfiguriert ist, dass er das Auftreffen von unerwünschten Photonen auf das photoelektrische Material (678) blockiert, wobei die unerwünschten Photonen eine Wellenlänge aufweisen, die ausgewählt ist aus der Gruppe bestehend aus:
Wellenlängen in einem oder mehreren Wellenlängenbereichen, die sich von der Bestrahlungswellenlänge unterscheiden;
Wellenlängen in einem oder mehreren Wellenlängenbereichen, die sich von einem Wellenlängenbereich unterscheiden, in dem ein oder mehrere Typen von Bio-Partikeln fluoreszieren; und
Wellenlängen in einem oder mehreren Wellenlängenbereichen, die sich von der Bestrahlungswellenlänge unterscheiden, und andere als ein Wellenlängenbereich, in dem ein oder mehrere Typen von Bio-Partikeln fluoreszieren.

7. Der Teilchendetektor nach Anspruch 5 oder 6, wobei das Gehäuse (120, 820, 920, 1020, 1120) eine Außenfläche und eine Innenfläche aufweist, und das photoelektrische Material (678) und der optische Filter (186, 886) gemäß einer Anordnung angeordnet sind, die ausgewählt ist aus der Gruppe bestehend aus:
das photoelektrische Material ist konform auf der Außenfläche angeordnet, und der optische Filter ist konform auf der Innenfläche angeordnet;
der optische Filter ist konform auf der Außenfläche angeordnet, und das photoelektrische Material ist konform auf dem optischen Filter angeordnet; und
das photoelektrische Material ist konform auf der Innenfläche angeordnet und der optische Filter ist konform auf dem photoelektrischen Material angeordnet.

8. Der Teilchendetektor nach einem der vorhergehenden Ansprüche, wobei das photoelektrische Material (678) ein photovoltaisches Material ist.

9. Der Teilchendetektor nach einem der vorhergehenden Ansprüche, wobei das photoelektrische Material (678) eine Vielzahl von nahe beieinander angeordneten photoelektrischen Einheiten (682) aufweist, wobei insbesondere die photoelektrischen Einheiten (682) in unterschiedlichen Winkelpositionen relativ zu der Längsachse (132, 932, 1032), an unterschiedlichen axialen Positionen relativ zu der Längsachse, oder sowohl an unterschiedlichen Winkelpositionen als auch axialen Positionen relativ zu der Längsachse angeordnet sind.

10. Der Teilchendetektor nach einem der vorhergehenden Ansprüche, wobei das photoelektrische Material (678) eine Vielzahl von photoelektrischen Materialien aufweist, wobei insbesondere die Vielzahl von photoelektrischen Materialien (678) eine Konfiguration aufweist, die ausgewählt ist aus der Gruppe bestehend aus:
die Vielzahl von photoelektrischen Materialien weist zwei oder mehr photoelektrische Materialien auf, die elektrisch in Reihe miteinander gekoppelt sind;
die Vielzahl von photoelektrischen Materialien weist zwei oder mehr photoelektrische Materialien auf, die elektrisch parallel miteinander gekoppelt sind;
die Vielzahl von photoelektrischen Materialien weist zwei oder mehr photoelektrische Materialien auf, die elektrisch voneinander isoliert sind; und
eine Kombination aus zwei oder mehreren der vorgenannten Möglichkeiten.

11. Der Teilchendetektor nach Anspruch 10, wobei die Vielzahl von photoelektrischen Materialien (682) eine Vielzahl von elektrisch isolierten photoelektrischen Materialien aufweist, und ferner eine Vielzahl von optischen Filtern (186, 886) aufweist, die jeweils zwischen einem oder mehreren der elektrisch isolierten photoelektrischen Materialien und der Längsachse (132, 932, 1032) angeordnet sind, so dass das Messlicht durch die optischen Filter hindurchgeht, wobei mindestens einer der optischen Filter so konfiguriert ist, dass er einen Wellenlängenbereich des Messlichts durchlässt, der sich von einem Wellenlängenbereich unterscheidet, der von den anderen optischen Filtern durchgelassen wird.

12. Der Teilchendetektor nach Anspruch 11, wobei der mindestens eine optische Filter (186, 886) so konfiguriert ist, dass er einen Wellenlängenbereich durchlässt, der Messlicht entspricht, das fluoreszierend von den Partikeln (112, 136) emittiert wird, während er einen Wellenlängenbereich, der von den Partikeln gestreutem Messlicht entspricht, blockiert.

13. Ein Verfahren zum Messen von Partikeln (112, 136) in einer Probenflüssigkeit, wobei das Verfahren aufweist:
Strömen der Probenflüssigkeit durch einen Detektionshohlraum (104, 804, 904, 1004, 1104), wobei der Detektionshohlraum (104, 804, 904, 1004, 1104) eine Hohlraumlänge entlang einer Längsachse (132, 932, 1032) aufweist, und wobei der Detektionshohlraum (104, 804, 904, 1004, 1104) von einem Gehäuse (120, 820, 920, 1020, 1120) umschlossen ist;
Richten eines Bestrahlungslichts durch den Detektionshohlraum (104, 804, 904, 1004, 1104) entlang der Längsachse (132, 932, 1032), um Partikel in der Probenflüssigkeit zu bestrahlen, wobei die Partikel in Reaktion auf die Bestrahlung Messlicht emittieren; und
Empfangen von Messlicht an einem photoelektrischen Material (678), das sich von den Partikeln (112, 136) in einer Vielzahl von Messlichtpfaden (116, 342, 344, 346), die relativ zu der Längsachse (132) abgewinkelt sind, ausbreitet,
**dadurch gekennzeichnet, dass**
das photoelektrische Material flexibel ist und es konform an dem Gehäuse (120, 820, 920, 1020, 1120) angeordnet ist und den Detektionshohlraum (104, 804, 904, 1004, 1104) umgibt, so dass sich das photoelektrische Material (678) entlang mindestens eines Teils der Hohlraumlänge um die Längsachse (132, 932, 1032) über eine Bogenlänge in einem Bereich von 30° bis 360° in einer Ebene orthogonal zu der Längsachse erstreckt.

## Revendications

1. Détecteur de particules (100, 800, 900, 1000, 1100), comprenant :
un boîtier (120, 820, 920, 1020, 1120) comprenant une entrée d'échantillon (152, 852, 952, 1052) et une sortie d'échantillon (154, 854, 954, 1054), et renfermant une cavité de détection (104, 804, 904, 1004, 1104) ayant une longueur de cavité le long d'un axe longitudinal (132, 932, 1032), dans lequel le boîtier définit un trajet d'écoulement pour un fluide d'échantillon à partir de l'entrée d'échantillon (152, 852, 952, 1052), à travers la cavité de détection (104, 804, 904, 1004, 1104) et jusqu'à la sortie d'échantillon (154, 854, 954, 1054) ; et
une source de lumière (124, 824, 924, 1024) configurée pour diriger une lumière d'irradiation le long de l'axe longitudinal (132, 932, 1032) vers des particules (112, 136) du fluide d'échantillon s'écoulant dans la cavité de détection (104, 804, 904, 1004, 1104) ;
un matériau photoélectrique flexible (678) disposé de manière conforme sur le boîtier (120, 820, 920, 1020, 1120) et entourant la cavité de détection (104, 804, 904, 1004, 1104) de sorte que, le long d'au moins une partie de la longueur de cavité, le matériau photoélectrique (678) s'étende autour de l'axe longitudinal (132, 932, 1032) sur une longueur d'arc dans une plage de 30° à 360° dans un plan orthogonal à l'axe longitudinal,
dans lequel le matériau photoélectrique (678) est configuré pour recevoir une lumière de mesure se propageant à partir des particules (112, 136) dans une pluralité de trajets de lumière de mesure (116, 342, 344, 346) inclinés par rapport à l'axe longitudinal (132, 932, 1032).

2. Détecteur de particules de la revendication 1, dans lequel la cavité de détection (104, 804, 904, 1004, 1104) a une configuration choisie dans le groupe constitué par :
la cavité de détection est généralement cylindrique, sphérique ou polygonale ;
au moins une partie de la cavité de détection a une aire de section transversale qui varie le long de l'axe longitudinal (132, 932, 1032) ;
la cavité de détection comprend une transition (1022) ayant une aire de section transversale qui augmente le long de l'axe longitudinal (132, 932, 1032) ;
la cavité de détection comprend une transition (1030) ayant une aire de section transversale qui diminue le long de l'axe longitudinal (132, 932, 1032) ;
la cavité de détection est exempte d'optiques de mise en forme de faisceau dans les trajets de lumière de mesure (116, 342, 344, 346) entre l'axe longitudinal (132, 932, 1032) et le matériau photoélectrique (678) ;
la cavité de détection comprend une première extrémité et une deuxième extrémité opposée sur l'axe longitudinal (132, 932, 1032), et la source de lumière (124, 824, 924, 1024) est configurée pour diriger la lumière d'irradiation de la première extrémité vers la deuxième extrémité ;
la cavité de détection comprend une première extrémité et une deuxième extrémité opposée sur l'axe longitudinal (132, 932, 1032), et la source de lumière (124, 824, 924, 1024) est montée sur le boîtier (120, 820, 920, 1020, 1120) au niveau de la première extrémité ; et
une combinaison de deux ou plus de ce qui précède.

3. Détecteur de particules de la revendication 1 ou 2, dans lequel la source de lumière (124, 824, 924, 1024) a une configuration choisie dans le groupe constitué par :
la source de lumière est configurée pour émettre la lumière d'irradiation sous la forme d'un faisceau cohérent, d'un faisceau collimaté ou à la fois d'un faisceau cohérent et collimaté ;
la source de lumière est configurée pour émettre la lumière d'irradiation ayant un diamètre de faisceau dans une plage de 0,4 mm à 4000 mm ;
la source de lumière est configurée pour émettre la lumière d'irradiation ayant une aire de section transversale dans une plage de 1% à 80% d'une aire de section transversale de la cavité de détection (104, 804, 904, 1004, 1104) ;
la source de lumière est configurée pour émettre la lumière d'irradiation à une longueur d'onde d'irradiation dans la plage ultraviolette, dans la plage visible ou dans la plage infrarouge ;
la source de lumière est configurée pour émettre la lumière d'irradiation à une longueur d'onde d'irradiation dans une plage de 250 à 1500 nm ;
la source de lumière est configurée pour émettre la lumière d'irradiation à une longueur d'onde d'irradiation dans une plage efficace pour induire l'autofluorescence dans un ou plusieurs type(s) de bioparticules ;
une combinaison de deux ou plus de ce qui précède.

4. Détecteur de particules de l'une des revendications précédentes, dans lequel la source de lumière (124, 824, 924, 1024) comprend une pluralité de sources de lumière, dans lequel au moins l'une des sources de lumière est configurée pour émettre la lumière d'irradiation à une longueur d'onde d'irradiation différente de celle des autres sources de lumière.

5. Détecteur de particules de l'une des revendications précédentes, comprenant un filtre optique (186, 886) positionné entre le matériau photoélectrique (678) et l'axe longitudinal (132, 932, 1032), dans lequel la lumière de mesure passe à travers le filtre optique.

6. Détecteur de particules de la revendication 5, dans lequel la source de lumière (124, 824, 924, 1024) est configurée pour émettre la lumière d'irradiation à une longueur d'onde d'irradiation, et le filtre optique (186, 886) est configuré pour empêcher les photons indésirables de heurter le matériau photoélectrique (678), les photons indésirables ayant une longueur d'onde choisie dans le groupe constitué par :
des longueurs d'onde dans une ou plusieurs plage(s) de longueurs d'onde autres que la longueur d'onde d'irradiation ; des longueurs d'onde dans une ou plusieurs plage(s) de longueurs d'onde autres qu'une plage de longueurs d'onde dans laquelle un ou plusieurs type(s) de bioparticules est/sont fluorescent(s) ; et
des longueurs d'onde dans une ou plusieurs plage(s) de longueurs d'onde autres que la longueur d'onde d'irradiation, et autres qu'une plage de longueurs d'onde dans laquelle un ou plusieurs type(s) de bioparticules est/sont fluorescent(s).

7. Détecteur de particules de la revendication 5 ou 6, dans lequel le boîtier (120, 820, 920, 1020, 1120) comprend une surface extérieure et une surface intérieure, et le matériau photoélectrique (678) et le filtre optique (186, 886) sont positionnés selon un agencement choisi dans le groupe constitué par :
le matériau photoélectrique est disposé de manière conforme sur la surface extérieure, et le filtre optique est disposé de manière conforme sur la surface intérieure ;
le filtre optique est disposé de manière conforme sur la surface extérieure, et le matériau photoélectrique est disposé de manière conforme sur le filtre optique ; et
le matériau photoélectrique est disposé de manière conforme sur la surface intérieure, et le filtre optique est disposé de manière conforme sur le matériau photoélectrique.

8. Détecteur de particules de l'une des revendications précédentes, dans lequel le matériau photoélectrique (678) est un matériau photovoltaïque.

9. Détecteur de particules de l'une des revendications précédentes, dans lequel le matériau photoélectrique (678) comprend une pluralité d'unités photoélectriques (682) positionnées à proximité les unes des autres, dans lequel en particulier les unités photoélectriques (682) sont agencées à des positions angulaires différentes par rapport à l'axe longitudinal (132, 932, 1032), à différentes positions axiales par rapport à l'axe longitudinal, ou à la fois à différentes positions angulaires et positions axiales par rapport à l'axe longitudinal.

10. Détecteur de particules de l'une des revendications précédentes, dans lequel le matériau photoélectrique (678) comprend une pluralité de matériaux photoélectriques, dans lequel en particulier la pluralité de matériaux photoélectriques (678) a une configuration choisie dans le groupe constitué par :
la pluralité de matériaux photoélectriques comprend deux matériaux photoélectriques ou plus couplés électriquement en série entre eux ;
la pluralité de matériaux photoélectriques comprend deux matériaux photoélectriques ou plus couplés électriquement en parallèle entre eux ;
la pluralité de matériaux photoélectriques comprend deux matériaux photoélectriques ou plus isolés électriquement les uns des autres ; et
une combinaison de deux ou plus de ce qui précède.

11. Détecteur de particules de la revendication 10, dans lequel la pluralité de matériaux photoélectriques (682) comprend une pluralité de matériaux photoélectriques électriquement isolés, et comprenant en outre une pluralité de filtres optiques (186, 886) respectivement positionnés entre un ou plusieurs des matériaux photoélectriques électriquement isolés et l'axe longitudinal (132, 932, 1032) de sorte que la lumière de mesure passe à travers les filtres optiques, dans lequel au moins l'un des filtres optiques est configuré pour laisser passer une plage de longueurs d'onde de la lumière de mesure différente d'une plage de longueurs d'onde traversant les autres filtres optiques.

12. Détecteur de particules de la revendication 11, dans lequel l'au moins un filtre optique (186, 886) est configuré pour laisser passer une plage de longueurs d'onde correspondant à une lumière de mesure émise par fluorescence par les particules (112, 136) tout en bloquant une plage de longueurs d'onde correspondant à une lumière de mesure diffusée par les particules.

13. Procédé de mesure de particules (112, 136) dans un fluide d'échantillon, le procédé comprenant :
l'écoulement du fluide d'échantillon à travers une cavité de détection (104, 804, 904, 1004, 1104), dans lequel la cavité de détection (104, 804, 904, 1004, 1104) a une longueur de cavité le long d'un axe longitudinal (132, 932, 1032) et dans lequel la cavité de détection (104, 804, 904, 1004, 1104) est enfermée par un boîtier (120, 820, 920, 1020, 1120) ;
la direction d'une lumière d'irradiation à travers la cavité de détection (104, 804, 904, 1004, 1104) le long dudit axe longitudinal (132, 932, 1032) pour irradier des particules dans le fluide d'échantillon, dans lequel les particules émettent une lumière de mesure en réponse à l'irradiation ; et
la réception, au niveau d'un matériau photoélectrique (678), d'une lumière de mesure se propageant à partir des particules (112, 136) dans une pluralité de trajets de lumière de mesure (116, 342, 344, 346) inclinés par rapport à l'axe longitudinal (132),
**caractérisé en ce que**
le matériau photoélectrique est flexible et il est disposé de manière conforme sur le boîtier (120, 820, 920, 1020, 1120) et entoure la cavité de détection (104, 804, 904, 1004, 1104) de sorte que, le long d'au moins une partie de la longueur de cavité, le matériau photoélectrique (678) s'étende autour de l'axe longitudinal (132, 932, 1032) sur une longueur d'arc dans une plage de 30° à 360° dans un plan orthogonal à l'axe longitudinal.
